(19) Europäisches Patentamt
European Patent Office
Office européen des brevets

(11) EP 4 223 741 A1

(12) EUROPEAN PATENT APPLICATION
published in accordance with Art. 153(4) EPC

(43) Date of publication:
09.08.2023 Bulletin 2023/32

(21) Application number: 21874553.7

(22) Date of filing: 29.09.2021

(51) International Patent Classification (IPC):
C07D 207/456 (2006.01) C07D 401/14 (2006.01)
A61K 31/537 (2006.01) A61P 35/00 (2006.01)
C07K 5/027 (2006.01) C07K 5/06 (2006.01)

(52) Cooperative Patent Classification (CPC):
A61K 31/4745; A61K 31/475; A61K 31/537;
A61K 31/5513; A61K 47/60; A61K 47/68;
A61P 35/00; C07D 207/456; C07D 401/14;
C07K 5/0205; C07K 5/06

(86) International application number:
PCT/CN2021/121825

(87) International publication number:
WO 2022/068898 (07.04.2022 Gazette 2022/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.09.2020 CN 202011046911

(71) Applicants:
- Mabwell (Shanghai) Bioscience Co., Ltd.
Shanghai 201210 (CN)
- Jiangsu Mabwell Health Pharmaceutical R&D Co., Ltd.
China Medical City
Taizhou, Jiangsu 225300 (CN)

(72) Inventors:
- ZHOU, Wei
Taizhou, Jiangsu 225300 (CN)
- ZHU, Huikai
Taizhou, Jiangsu 225300 (CN)
- WANG, Zhenzhen
Taizhou, Jiangsu 225300 (CN)
- XU, Hui
Taizhou, Jiangsu 225300 (CN)
- TAN, Xiaoding
Taizhou, Jiangsu 225300 (CN)

(74) Representative: Schiweck Weinzierl Koch
Patentanwälte Partnerschaft mbB
Ganghoferstraße 68 B
80339 München (DE)

# (54) PREPARATION METHOD FOR BIS-SUBSTITUTED BRIDGING ANTIBODY-DRUG CONJUGATE

(57) A preparation method for a bis-substituted bridging antibody-drug conjugate, comprising: hydrolyzing an antibody coupling product obtained by coupling an antibody to a compound as represented by formula I. Also provided is an antibody coupling product obtained by the preparation method.

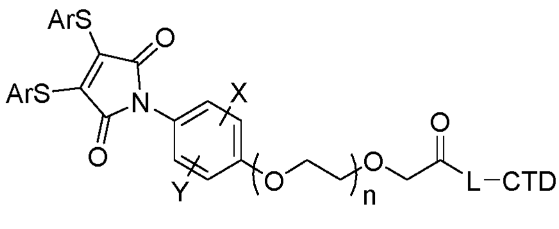

I



Processed by Luminess, 75001 PARIS (FR)

## Description

### CROSS-REFERENCE TO RELATED APPLICATIONS

The present patent application claims the priority benefit of Chinese Patent Application No. CN202011046911.X filed on 29 September 2020, which is incorporated herein by reference in its entirety.

### TECHNICAL FIELD

The present invention belongs to the field of antibody drug preparation processes, and particularly relates to a method for preparing an antibody-drug conjugate using a disubstituted maleimide linker.

### BACKGROUND OF THE INVENTION

An antibody-drug conjugate (ADC) is a class of novel therapeutics for tumor treatment which is generally composed of an antibody or antibody-like ligand, a small molecule drug, and a linker coupling the two together. It combines the anti-tumor activity of the small molecule drug with the high selectivity and stability and the good pharmacokinetic characteristics of the antibody or antibody-like ligand, and currently is an attentive hotspot in the field of tumor treatment.

The conjugation techniques and the preparation processes are very critical for developing ADCs. In order to ensure the clinical safety and effectiveness of such medicaments, it is not only necessary to achieve a stable linkage between the drug and the antibody, but also to minimize the heterogeneity of the ADC products obtained. At present, the third generation conjugation technology to generate ADCs is generally applied and mainly includes three types, namely the site-specific conjugation technology via unnatural amino acids, the site-specific conjugation technology by enzyme catalysis and the site-specific conjugation technology through chemical modification. The first two technologies usually require special modifications on the antibodies, so that the site-specific conjugation technology through chemical modification is a more generally used technology for the preparation of antibody-drug conjugates for common antibody molecules. The technology mainly utilizes a linker of a specific structure and a coupling process adapted to the linker to realize the site-specific conjugation. For example, patent application CN201380025774.3 discloses a preparation scheme for ADCs using a bridging linker; and patent application CN201310025021.4 discloses a preparation scheme for ADCs using a tridentate linker.

MABWELL (SHANGHAI) BIOSCIENCE CO., LTD. and its subsidiary JIANGSU MABWELL HEALTH PHARMACEUTICAL R&D CO., LTD. developed a new technology of linkers, based which they provided a novel disubstituted maleimide linker and an ADC prepared using the same (see WO2018/095422A1). The obtained ADCs, based on the disulfide bond bridging of disubstituted maleimide, have better stability, are less prone to sulfhydryl-ether exchange in vivo, and have good in vivo and in vitro drug activities. However, it has been found that the ADCs prepared following the process disclosed in WO2018/095422A1 tend to have poor product homogeneity, which presumably is caused by relatively severe shedding of small molecule drugs. From the perspective of drug safety, the shedding of small molecule drugs may lead to non-targeted toxicity, and bring a major safety problem to drug development.

Therefore, there is a need in the art to develop a site-specific conjugation process for preparing medicaments, which is compatible with disubstituted maleimide linkers and also is simple, efficient, stable, and has practical significance.

### SUMMARY OF THE INVENTION

It has been experimentally proved that ADCs products prepared following the process and conditions disclosed in WO2018/095422A1 still cannot meet the safety, effectiveness and stability and other quality requirements for clinical medications, and cannot be commercialized produced either. In order to solve the problem, the object of the present disclosure is to provide a novel preparation method for a disubstituted bridged antibody-drug conjugate.

The present disclosure provides the following technical solutions.

In one aspect, the present disclosure provides a preparation method for a disubstituted bridged antibody-drug conjugate, comprising: subjecting an antibody conjugation product obtained by conjugating an antibody to a compound represented by formula I to hydrolysis:

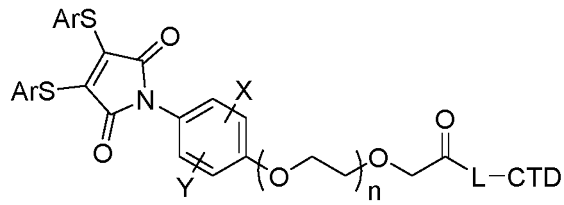

I

In formula I, Ar is phenyl or substituted phenyl, and the substituent in the substituted phenyl is selected from the group consisting of alkyl (e.g., $C_1$-$C_6$ alkyl, preferably $C_1$-$C_3$ alkyl, more preferably methyl), alkoxy (e.g., $C_1$-$C_6$ alkoxy, preferably $C_1$-$C_3$ alkoxy, more preferably methoxy), halogen (F, Cl, Br or I), ester, cyano and -C(O)$NR_1R_2$-, in which $R_1$ and $R_2$ are independently selected from the group consisting of a chemical bond, H and alkyl (e.g., $C_1$-$C_6$ alkyl, preferably $C_1$-$C_3$ alkyl, more preferably methyl), or $R_1$ and $R_2$ form a 5-7 membered heterocyclic ring with one or more heteroatoms selected from the group consisting of O, N and S;

X and Y are independently hydrogen, halogen (F, Cl, Br or I), trifluoromethyl or alkoxy (e.g., $C_1$-$C_6$ alkoxy, preferably $C_1$-$C_3$ alkoxy, preferably methoxy);
n is any integer between 1 and 24;
L-CTD is a cytotoxic drug and a connection release structure applied in an antibody-drug conjugate, in which L is, for example, selected from the group consisting of Val-Ala-PAB (VA-PAB), Val-Cit-PAB (VC-PAB), Phe-Lys-PAB, and MAC glucuronide phenol linker; and CTD is, for example, a cytotoxic drug, preferably a tubulin inhibitor (e.g., tubulin inhibitors MMAE, DM1, DM4, Tublysin); a topoisomerase inhibitor (e.g., topoisomerase inhibitors SN38, Exatecan and derivatives thereof) or a DNA binding agent (e.g., DNA binding agent PBD and derivatives thereof). PAB is p-aminobenzyloxycarbonyl.

Preferably, $R_1$ and $R_2$ are independently selected from the group consisting of H and $C_1$-$C_3$ alkyl; or $R_1$ and $R_2$ form a 6-membered heterocyclic ring with one or more heteroatoms selected from the group consisting of O and N, preferably morpholine.

More preferably, Ar is phenyl, 4-methylformamido-substituted phenyl

( 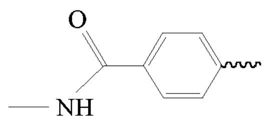 ),

or 4-formylmorpholine-substituted phenyl

( 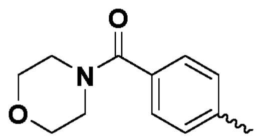 ).

Preferably, X and Y are independently hydrogen, fluoro, trifluoromethyl or methoxy; more preferably, X and Y are independently hydrogen, or X and Y are independently at the meta position on the phenyl ring relative to the maleimide.

Preferably, n is any integer between 1 and 10, preferably between 3 and 5.

Preferably, L-CTD is VC-PAB-MMAE or VC-seco-DUBA.

Preferably, the hydrolysis is performed before or after purification of the antibody conjugation product.

Preferably, the hydrolysis comprises: heating the antibody conjugation product in a hydrolysis buffer at pH 7.4-9.0 at 25-45 °C for 1-24 hours. The hydrolysis buffer may comprise one or more selected from the group consisting of sodium dihydrogen phosphate, disodium hydrogen phosphate, dipotassium hydrogen phosphate, potassium dihydrogen phosphate, citric acid, glycine, tromethamine, arginine hydrochloride, hydrochloric acid, phosphoric acid, sodium hydroxide, and potassium hydroxide. Preferably, the hydrolysis buffer is a sodium dihydrogen phosphate - disodium hydrogen phosphate buffer, a potassium dihydrogen phosphate - dipotassium hydrogen phosphate buffer, or a tromethamine buffer.

Preferably, the concentration of the antibody conjugation product in the hydrolysis buffer is 2-30 mg/mL, preferably 5-20 mg/mL.

More preferably, the hydrolysis comprises: heating the antibody conjugation product in the hydrolysis buffer at 25-35 °C for 1-6 hours, preferably 1-3 hours. Preferably, the hydrolysis buffer is at pH 7.5-8.5, preferably pH 7.8. For example, the hydrolysis buffer is a sodium dihydrogen phosphate - disodium hydrogen phosphate buffer at pH 7.5-8.5.

According to one particular embodiment of the present invention, the hydrolysis buffer is: 50 mM sodium dihydrogen phosphate - disodium hydrogen phosphate buffer, pH 7.8; 50 mM sodium dihydrogen phosphate - disodium hydrogen phosphate buffer + 3% arginine, pH 7.8; 50 mM potassium dihydrogen phosphate - dipotassium hydrogen phosphate buffer, pH 7.8; or 50 mM tromethamine - hydrochloric acid buffer, pH 7.8.

Preferably, the antibody is an IgG antibody, preferably IgG1 antibody.

According to one particular embodiment of the present invention, the antibody conjugation product is prepared according to the method disclosed in WO2018/095422A1.

Specifically, the preparation method provided by the present disclosure comprises the following steps:

a. Antibody reduction: adding a reducing agent in a amount of ≥ 5.5 molar equivalents of the antibody to a buffer containing the antibody in a concentration of 5-30 mg/mL, and reacting the reducing agent and the antibody at 30-40 °C for 1.5-2 hours, wherein the reducing agent is one or more selected from the group consisting of TCEP, DTT, 2-MEA, and DTBA;

b. Antibody conjugation: displacing the reduced antibody obtained in step a to a buffer at pH 6.5-7.6 in which the antibody is diluted to a concentration of 3.5-15 mg/mL to obtain a diluted antibody solution; adding a compound represented by formula I in a amount of 4.5-6.5 molar equivalents of the antibody dissolved in an organic co-solvent to the diluted antibody solution, such that the volume of the organic co-solvent accounts for 5-30% of the volume of the reaction system, and then stirring and reacting the reaction system at 15-35 °C for ≥ 0.5 hours, wherein the organic co-solvent is one or more selected from the group consisting of DMA, DMSO, DMF, and ACN;

c. Hydrophobic chromatography: subjecting the antibody conjugation product obtained to purification through hydrophobic chromatography using hydrophobic filler, wherein the hydrophobic filler is Butyl Sepharose HP (GE), Capto Phenyl ImpRes (GE), Toyopearl Butyl 650M (TOSOH) or Butyl Sepharose 4 FF (GE), and the start buffer for the hydrophobic chromatography contains sodium chloride or ammonium sulfate.

The preparation method further comprises the following step after step b or after step c:
d. Hydrolysis: displacing the antibody conjugation product into a hydrolysis buffer at pH 7.4-9.0, and heating the same therein at 25-45 °C for 1-24 hours. The hydrolysis buffer may comprise one or more selected from the group consisting of sodium dihydrogen phosphate, disodium hydrogen phosphate, dipotassium hydrogen phosphate, potassium dihydrogen phosphate, citric acid, glycine, tromethamine, arginine hydrochloride, hydrochloric acid, phosphoric acid, sodium hydroxide, and potassium hydroxide.

In the method according to the present invention, the displacement in steps a, b and d is performed through gel chromatography, centrifugal filtration, or ultrafiltration separation.

In step a in the preparation method according to the present invention:

preferably, the reducing agent is TCEP or DTT;
preferably, the reducing agent is in an amount of 6.5-10 molar equivalents of the antibody;
preferably, the reducing agent is added in a form of an aqueous solution at a concentration of 5-15 mg/mL, preferably 10 mg/mL;
preferably, the antibody is an IgG antibody, preferably IgG1 antibody;
preferably, the concentration of the antibody is 10-15 mg/mL, preferably 12 mg/mL;
preferably, the buffer is a phosphate buffer at pH 6.5-7.6. According to one particular embodiment of the present invention, the buffer is 50 mM phosphate buffer, pH 7.4.

In step b in the preparation method according to the present invention:

preferably, the buffer is at pH 7.2-7.4; preferably, the buffer is a phosphate buffer or a phosphate-EDTA buffer at pH 7.2-7.4. According to one particular embodiment of the present invention, the buffer is 50 mM sodium dihydrogen phosphate - disodium hydrogen phosphate + 100 mM NaCl + 2 mM EDTA buffer, pH 7.2-7.4;
preferably, when displaced, the antibody is diluted to a concentration of 3.5-10 mg/mL;
preferably, the compound represented by formula I is in an amount of 5.0-6.0 molar equivalents of the antibody;
preferably, the organic co-solvent is DMA, DMSO, or ACN;
preferably, the organic co-solvent is added to account for 6-30% of the volume of the reaction system;
preferably, the reaction system is stirred and reacted at 15-35 °C for 0.5-1 hour.

In step c in the preparation method according to the present invention:

preferably, the start buffer contains ammonium sulfate. According to one embodiment of the present invention, the concentration of ammonium sulfate is preferably 0.45 mol/L;
preferably, the start buffer is a phosphate buffer containing ammonium sulfate, and a phosphate buffer is used as an eluent;
more preferably, the phosphate buffer is 0-100 mM disodium hydrogen phosphate - sodium dihydrogen phosphate buffer at pH 7.4-8.0.

In step d in the preparation method according to the present invention:

preferably, the concentration of the antibody conjugation product in the hydrolysis buffer is 2-30 mg/mL, preferably 5-20 mg/mL;
preferably, the hydrolysis buffer is a sodium dihydrogen phosphate - disodium hydrogen phosphate buffer, a potassium dihydrogen phosphate - dipotassium hydrogen phosphate buffer or a tromethamine buffer;
preferably, the hydrolysis comprises: heating the antibody conjugation product in the hydrolysis buffer at 25-35 °C for 1-6 hours, preferably 1-3 hours. Preferably, the hydrolysis buffer is at pH 7.5-8.5, preferably pH 7.8. For example, the hydrolysis buffer is a sodium dihydrogen phosphate - disodium hydrogen phosphate buffer at pH 7.5-8.5.

According to one particular embodiment of the present invention, the hydrolysis buffer is: 50 mM sodium dihydrogen phosphate - disodium hydrogen phosphate buffer, pH 7.8; 50 mM sodium dihydrogen phosphate - disodium hydrogen phosphate buffer + 3% arginine, pH 7.8; 50 mM potassium dihydrogen phosphate - dipotassium hydrogen phosphate buffer, pH 7.8; or 50 mM tromethamine - hydrochloric acid buffer, pH 7.8.

In another aspect, the present disclosure provides a disubstituted bridged antibody-drug conjugate obtained using the method of the present invention.

WO2018/0954221A1 describes the following preparation process for ADCs:

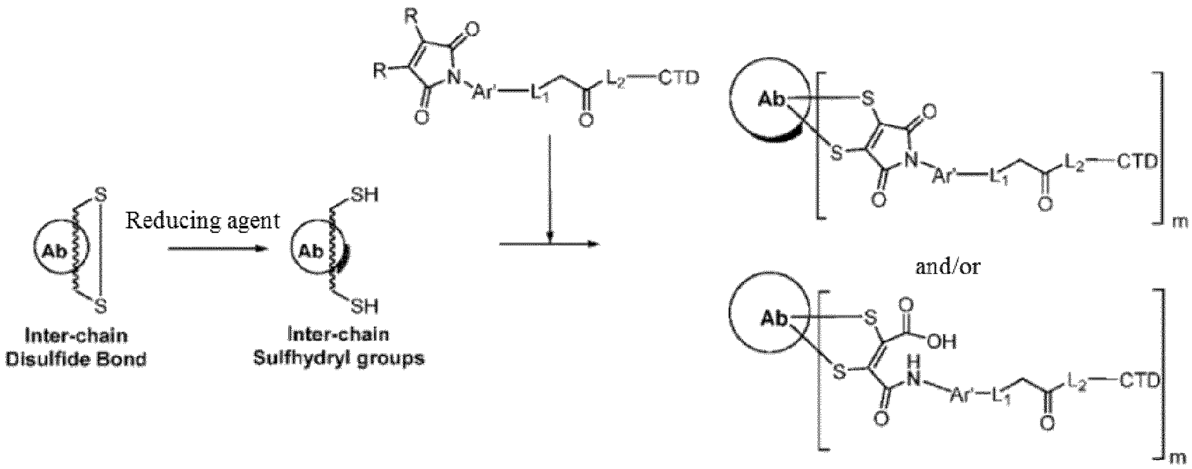


Thereby a mixed ADC product of two structures was prepared. It has been experimentally shown that the mixed product has poor stability in vitro and in vivo and has serious infusion-related response during administration. This may be due to the shedding of small molecule drug from the antibody, which may have varying degrees of negative impact on the efficacy and safety of the product.

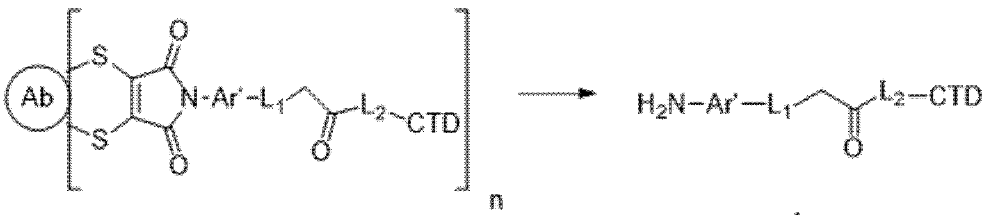

To solve this problem, a hydrolysis step is introduced in the method according to the present invention, after the step of antibody conjugation to obtain the ADC, and may be performed before or after column chromatography

purification of the ADC. For example, the product of each of the steps b and c can be incubated in a hydrolysis buffer at pH 8.0 ± 0.5 (e.g., 50 mM sodium dihydrogen phosphate - disodium hydrogen phosphate buffer), so that one molecule of maleimide undergoes an addition reaction with one molecule of water to produce ring-opened carboxylic acid compound, thereby obtaining a uniform ring-opened ADC product.

Experiments proved that after hydrolysis treatment, the structure of the ADC product was more uniform, and the shedding of the small-molecule drugs was obviously reduced during incubation of the uniform ADC product in plasma at 37 °C.

In addition, reaction parameters utilized in the steps of antibody reduction, conjugation and chromatography purification operations were further screened, with which a higher purity of the final ADC product was achieved (purity greater than 95% as shown by NR-CE-SDS). Meanwhile, experiments proved that the process of the method can be smoothly scaled-up, and conjugated samples obtained by a scaled-up process have substantially the same physicochemical properties as conjugated samples obtained by a laboratory scale.

## BRIEF DESCRIPTION OF THE DRAWINGS

Embodiments of the present invention are described in detail below with reference to the attached figures, in which:

FIG. 1 shows the characterization results of the ADC product samples obtained with different antibodies using the preparation method according to the present invention in Example 7, in which:
1A to 1C show the characterization results of the products obtained with Pertuzumab, via HIC-HPLC, NR-CE-SDS and SEC-HPLC respectively.

Fig. 2 shows the characterization results of the ADC product samples of groups A and B subjecting to hydrolysis or not in Example 8, in which:

2A to 2B show the characterization results of the samples of groups A and B via HIC-HPLC, respectively;
2C to 2D show the characterization results of the samples of groups A and B via SEC-HPLC, respectively;
2E to 2F show the characterization results of the samples of groups A and B via NR-CE-SDS, respectively.

## DETAILED DESCRIPTION OF PREFERRED EMBODIMENTS

The method according to the present invention provides an ADC product of a more stable quality and more homogeneity as follows:

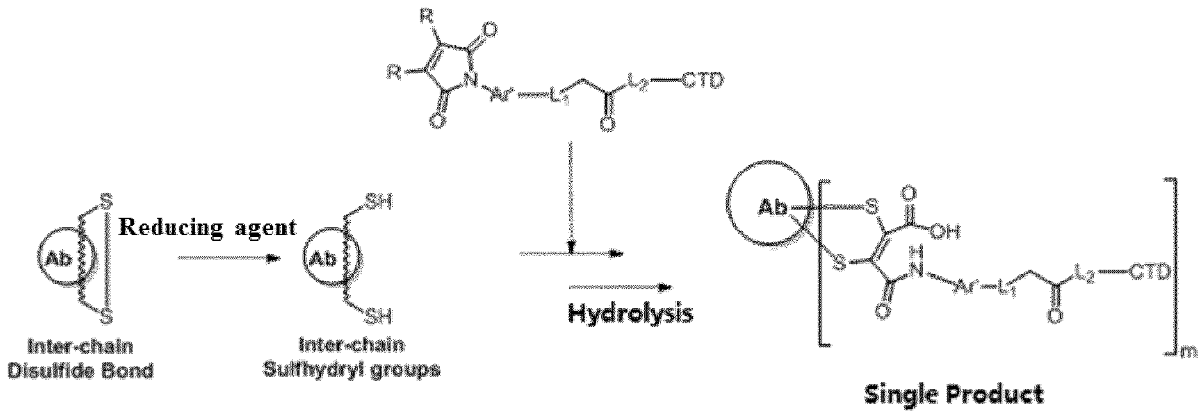


The "equivalent" mentioned in the present disclosure refers to a molar equivalent with respect to the antibody.

The "compound represented by formula I" mentioned in the present disclosure is used interchangeably herein with "small molecule" or "small molecule compound", and its structure is exemplified as follows:

Molecules of A series:

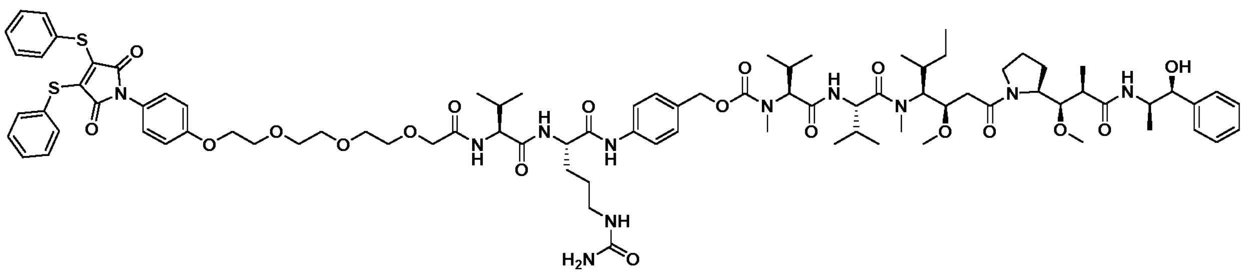

A-1

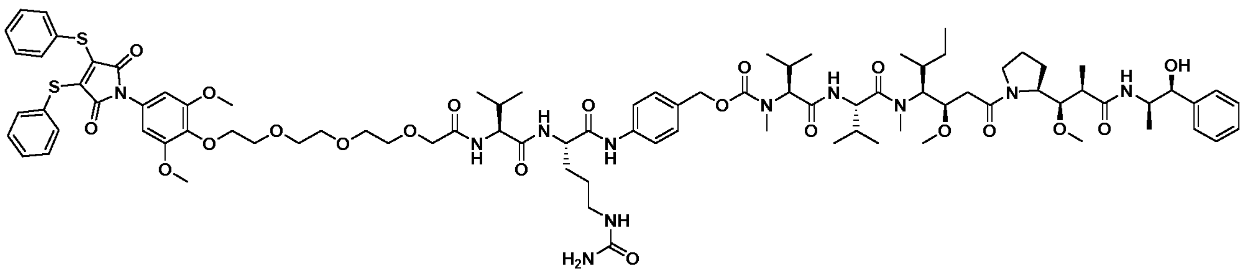

A-2

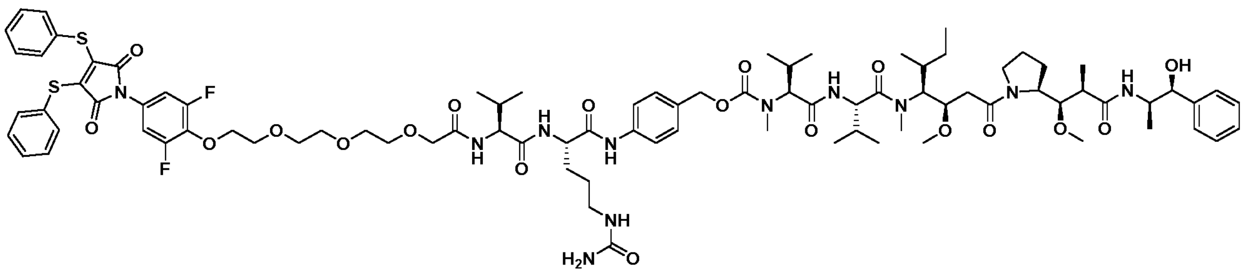

A-3

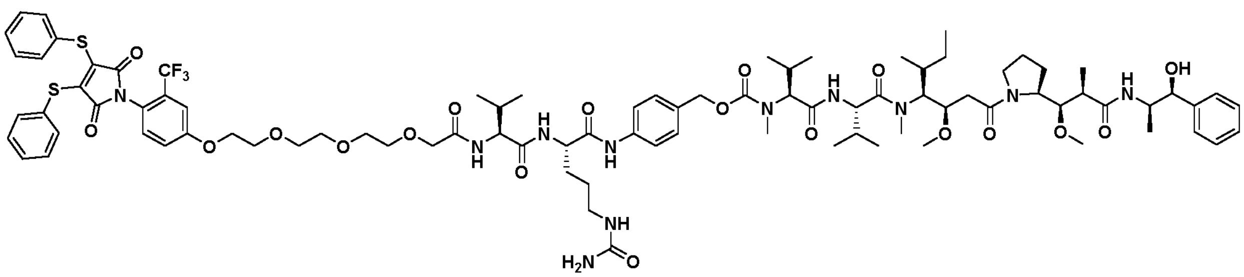

A-4

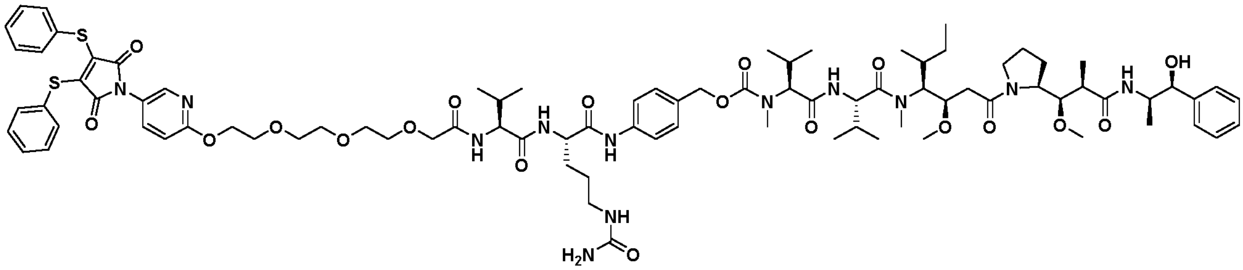

A-5

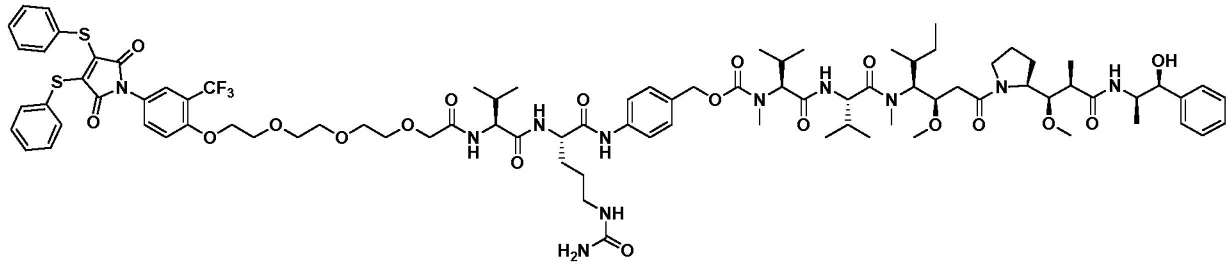

A-6

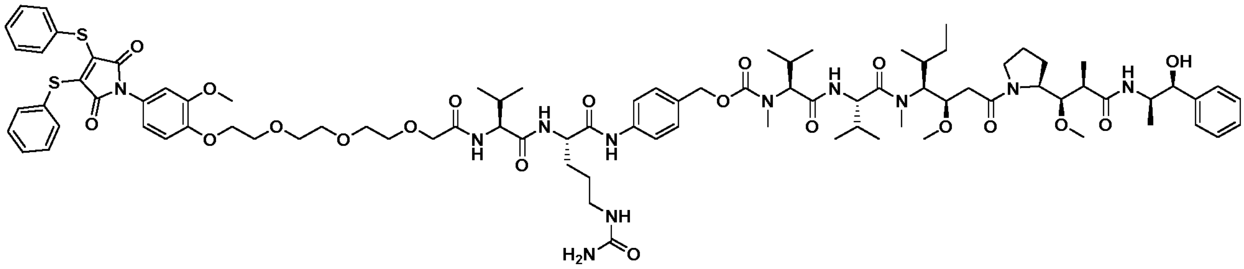

A-7

Molecules of B series:

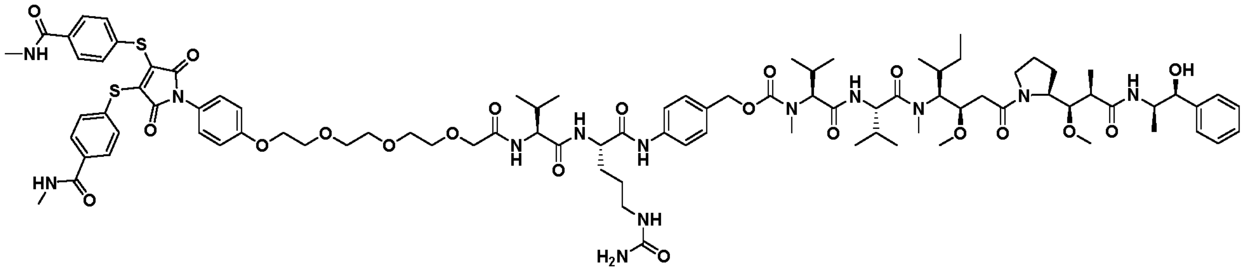

B-1

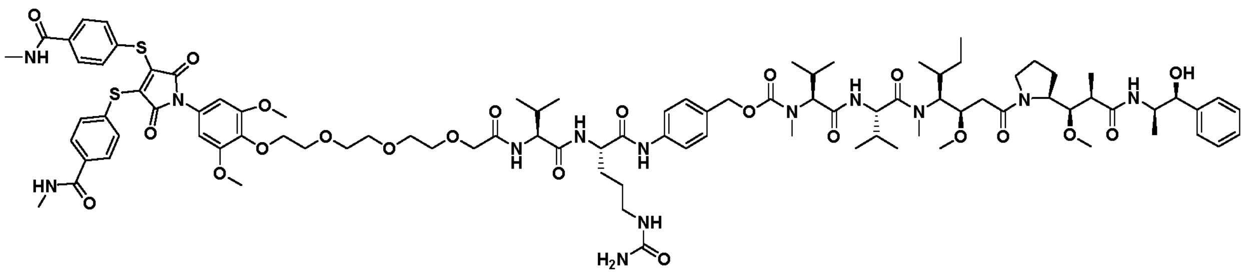

B-2

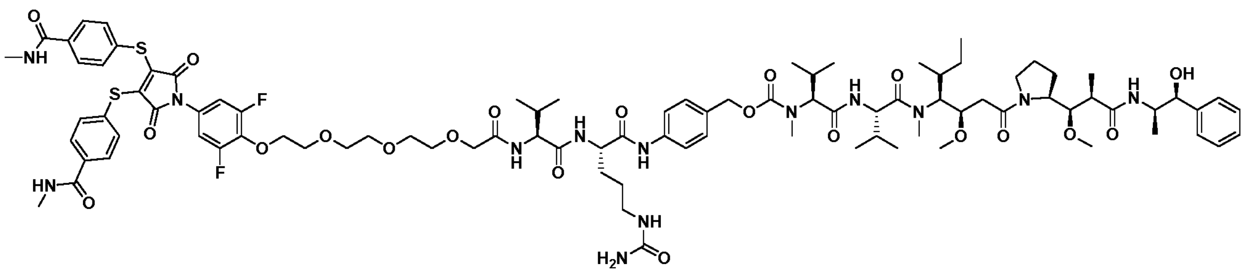

B-3

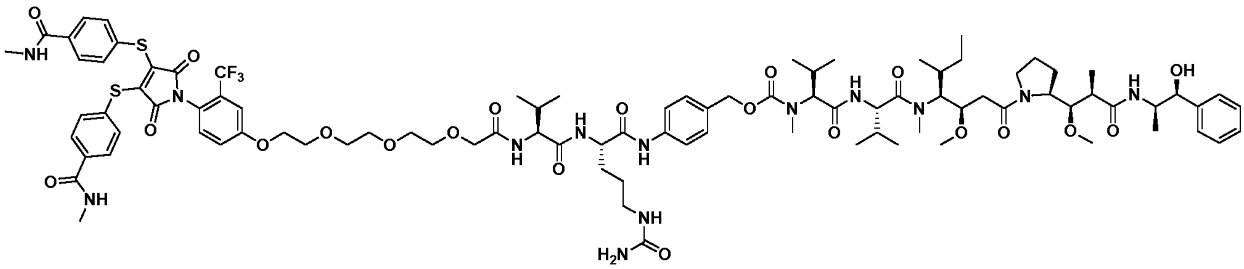

B-4

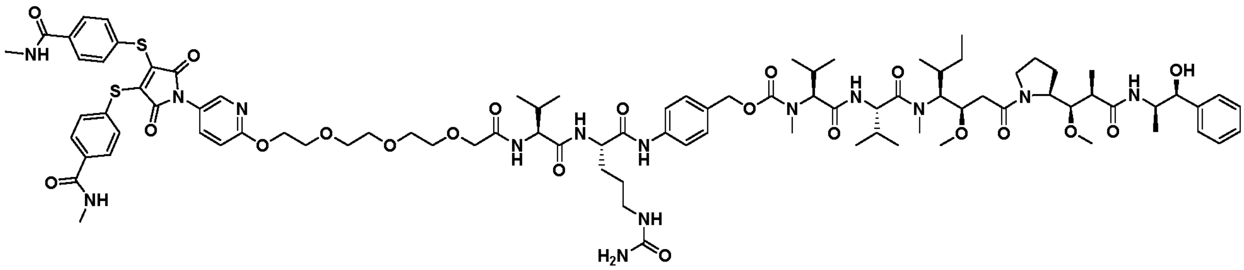

B-5

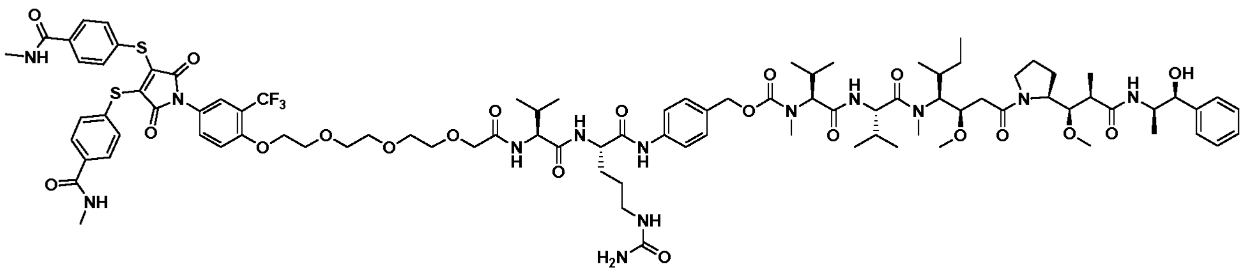

B-6

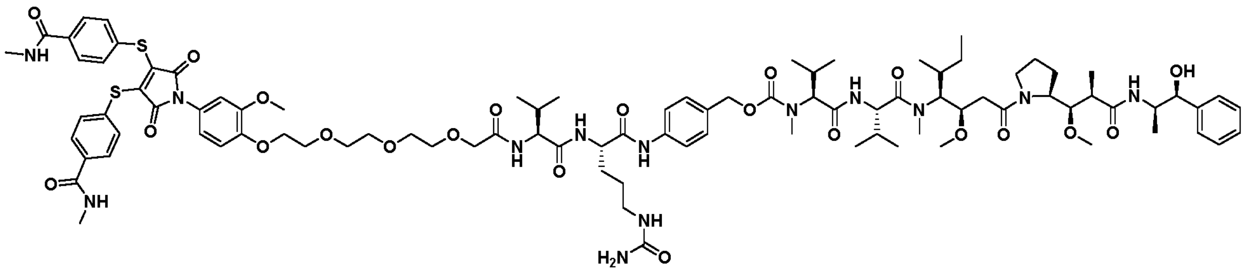

B-7

Molecules of C series:

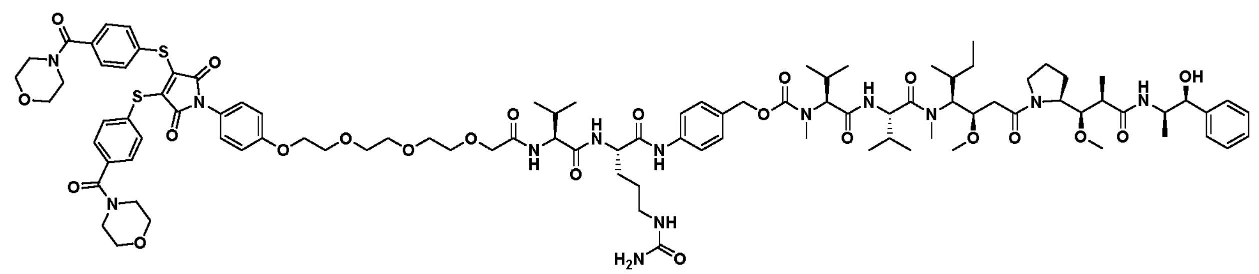

C-1

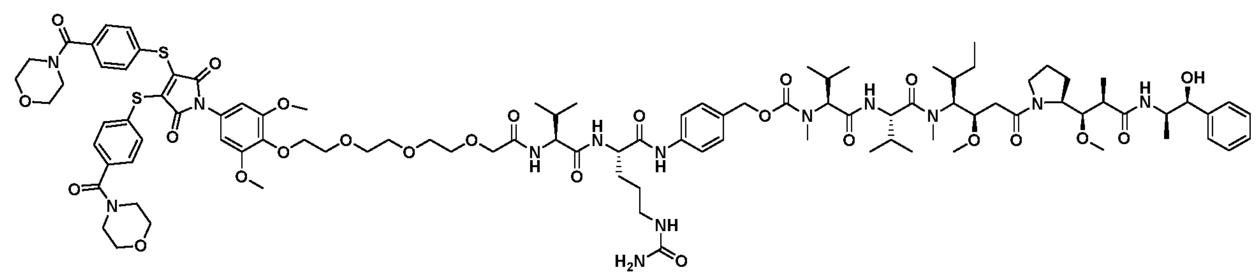

C-2

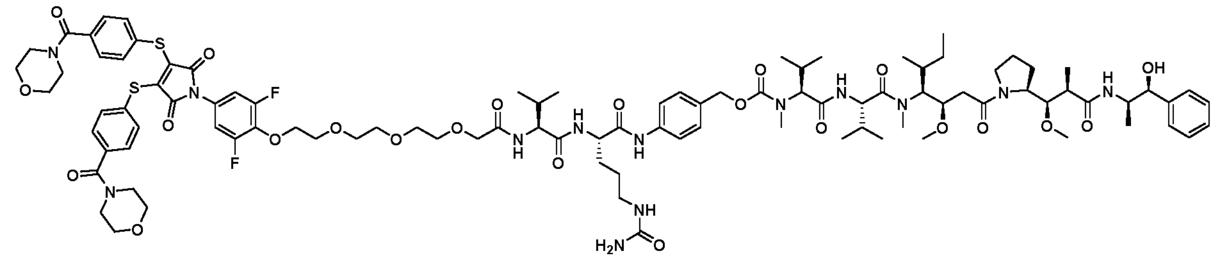

C-3

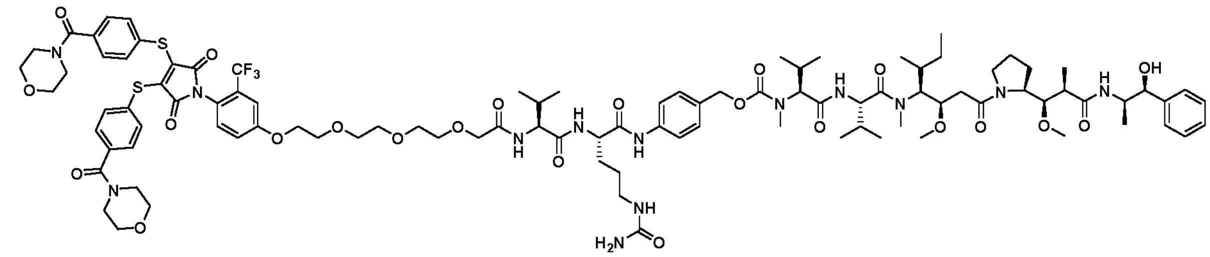

C-4

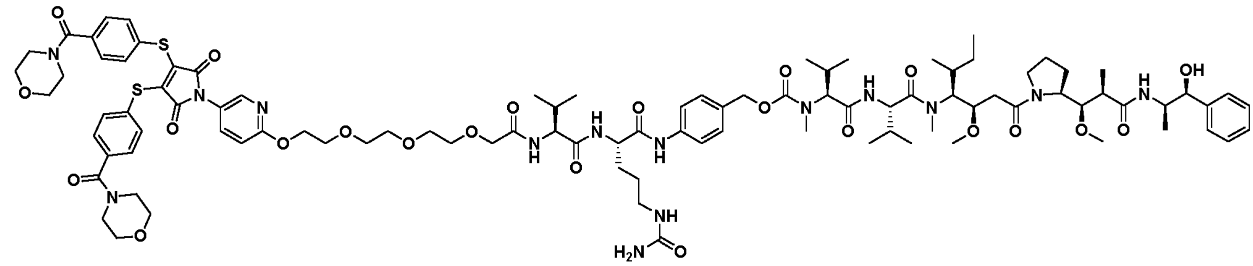

C-5

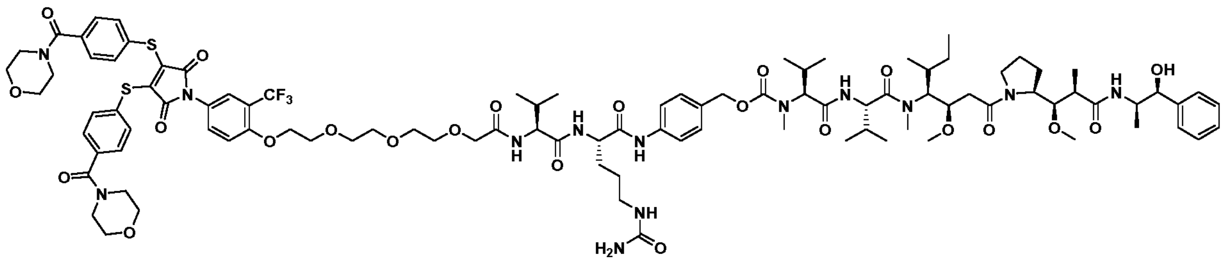

C-6

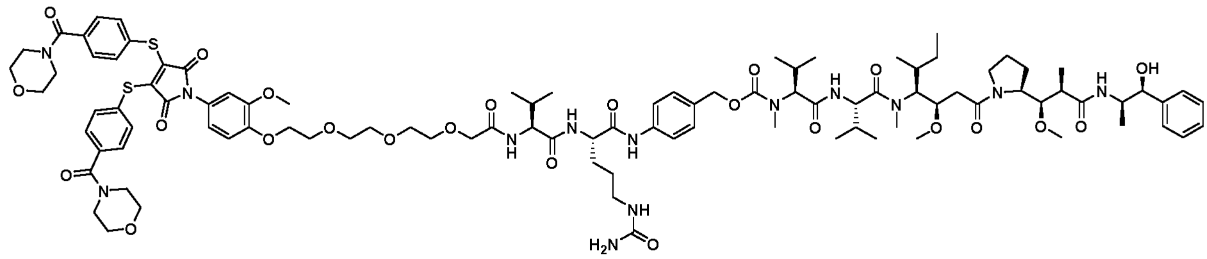

C-7

Molecules of D series:

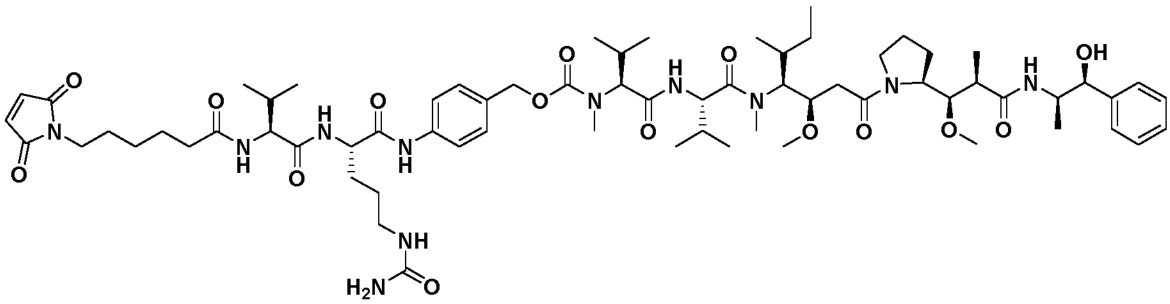

D-1

"PB" used in the present disclosure refers specifically to a sodium phosphate buffer containing sodium dihydrogen phosphate - disodium hydrogen phosphate as the major ingredients. Sodium dihydrogen phosphate - disodium hydrogen phosphate buffers at different pH values are typically formulated using sodium dihydrogen phosphate and disodium hydrogen phosphate solutions at the same concentration.

General detection methods are as follows:

(1) Hydrophobic interaction chromatography (HIC-HPLC):

(a1) Hydrophobic chromatography HIC-HPLC used to determine the DAR values of site-specific ADCs:

Sample preparation: the sample was diluted to 2.0 mg/ml with the mobile phase B, and then was centrifuged at 12000 rpm for 10 min; and the supernatant was taken for HPLC analysis.

Chromatographic column: Sepax Proteomix HIC Butyl-NP5, 5 μm, 4.6 mm×35 mm;

Mobile phase A: 0.025 M phosphate +1.2 M ammonium sulphate (pH 7.0);

Mobile phase B: 0.025 M phosphate (pH 7.0);

Mobile phase C: 100% IPA;

Flow rate: 0.8 mL/min;

Detection wavelength: 280 nm;

Column temperature: 30 °C;

Loading volume: 20 μL;

Chromatographic gradient used for HIC analysis:

| Time (min) | Mobile phase A (%) | Mobile phase B (%) | Mobile phase C (%) |
|---|---|---|---|
| 0 | 100 | 0 | 0 |
| 1 | 100 | 0 | 0 |
| 12 | 0 | 80 | 20 |
| 16 | 0 | 80 | 20 |
| 17 | 100 | 0 | 0 |
| 21 | 100 | 0 | 0 |

Formula for DAR calculation:

$$\text{DAR} = \Sigma\ (\text{weighted peak area})/100,\ \text{i.e., DAR} = (\text{D0 peak area ratio} \times 0 + \text{D1 peak area ratio} \times 1 + \text{D2 peak area ratio} \times 2 + \text{D3 peak area ratio} \times 3 + \text{D4 peak area ratio} \times 4 + \text{D5 peak area ratio} \times 5 + \text{D6 peak area ratio} \times 6 + \text{D7 peak area ratio} \times 7 + \text{D8 peak area ratio} \times 8)/100.$$

Note: DAR values of the molecules of A, B, and C series were determined by the analysis method provided in (a1).

(a2) Hydrophobic chromatography HIC-HPLC used to determine the DAR values of site-specific ADCs:

Sample preparation: the sample was diluted to 3.0 mg/ml with the mobile phase B, and then was centrifuged at 12000 rpm for 10 min; and the supernatant was taken for HPLC analysis. Chromatographic column: Sepax Proteomix HIC Butyl-NP5, 5 μm, 4.6 mm×35 mm;

Mobile phase A: 125 mM PB + 2.5 M $(NH_4)_2SO_4$ (pH 6.7);

Mobile phase B: 125 mM PB (pH6.7);

Mobile phase C: IPA;

Mobile phase D: $H_2O$;

Flow rate: 0.5 mL/min;

Detection wavelength: 280 nm;

Column temperature: 25 °C;

Loading volume: 10 μL;

Chromatographic gradient used for HIC analysis:

| Time (min) | Mobile phase A (%) | Mobile phase B (%) | Mobile phase C (%) | Mobile phase D (%) |
|---|---|---|---|---|
| 0 | 80 | 0 | 0 | 20 |
| 20 | 5 | 45 | 3 | 47 |
| 22 | 0 | 50 | 3 | 47 |
| 22.1 | 80 | 0 | 0 | 20 |
| 30 | 80 | 0 | 0 | 20 |

DAR values were calculated as described in (a1).

Note: DAR values of the molecules of L series were determined by the analysis method provided in (a2).

(a3) Hydrophobic chromatography HIC-HPLC used to determine the DAR values of randomly conjugated ADCs:

Sample preparation: the sample was diluted to 2.0 mg/ml with the mobile phase B, and then was centrifuged at 12000 rpm for 10 min; and the supernatant was taken for HPLC analysis.

Chromatographic column: TOSOH Butyl NPR, 2.5 μm, 4.6 mm×100 mm;

Mobile phase A: 125 mM PB + 2.5 M $(NH_4)_2SO_4$ (pH 6.8);

Mobile phase B: 125 mM PB (pH 6.8);

Mobile phase C: 100% IPA;

Mobile phase D: $H_2O$;

Flow rate: 0.7 mL/min;

Detection wavelength: 280 nm;

Column temperature: 30 °C;

Loading volume: 10 μL;

Chromatographic gradient used for HIC analysis:

| Time (min) | Mobile phase A (%) | Mobile phase B (%) | Mobile phase C (%) | Mobile phase D (%) |
|---|---|---|---|---|
| 0 | 50 | 0 | 5 | 45 |
| 10 | 0 | 50 | 5 | 45 |
| 20 | 0 | 50 | 5 | 45 |
| 20.1 | 50 | 0 | 5 | 45 |
| 35 | 50 0 | 5 | 45 | |

DAR values were calculated as described in (a1).
Note: DAR values of the molecules of D series were determined by the analysis method provided in (a3).
D3%, D4%, and D5% mentioned herein represent percentages the ADCs having DAR values of 3, 4, and 5 accounted for respectively.

(2) D-SEC:

Chromatographic column: Zenix-C SEC-300;
Mobile phase: water:acetonitrile = 65:35 (both containing 0.1% TFA and 0.1% FA);
Flow rate: 0.2 mL/min;
Column temperature: 25 °C;
Detection wavelength: 280 nm;
Elution gradient: isocratic elution.

(3) Non-reducing capillary electrophoresis (NR-CE-SDS):

Sample treatment: the sample was diluted to 2.0 mg/ml (sample buffer: 40 mM PB, 1% SDS, pH 6.5); 50 μL of the diluted sample was mixed with 45 μL of the sample buffer + 5 μL of IAM (0.5 M) thoroughly, and the mixture obtained was incubated in a water bath at 70 °C for 10 min; and the supernatant was taken for analysis.
The analysis conditions were as follows:
Capillary activation: the capillary was rinsed with 0.1 mol/L sodium hydroxide for 10 min, 0.1 mol/L hydrochloric acid for 5 min and water for injection for 2 min respectively at 20 psi; and then rinsed with SDS-MW-Gel Buffer for 10 min at 70 psi; afterwards, pre-separation was performed for 10 min at 20 psi, at a column temperature of 25 °C and a voltage of -15 kv. The program was run: at 70 psi, the capillary was rinsed with 0.1 mol/L sodium hydroxide for 3 min, 0.1 mol/L hydrochloric acid for 1 min and water for injection for 1 min respectively; then the gel was poured for 10 min, the sample was loaded for 40 s at -5 kv, and the separation program was run (-18 kv, 20 psi). The column temperature was set to 25 °C, the voltage for separation was set to -15 kv, and the separation was performed for 40 min.

(4) Size exclusion chromatography (SEC-HPLC):

Sample treatment: the sample was diluted to 2.0-3.0 mg/ml with the mobile phase, and then was centrifuged at 12000 rpm for 10 min; and the supernatant was taken for analysis.
Chromatographic column: TOSOH, TSKgel G3000$SW_{XL}$, 5 μm, 7.8 mm×300 mm;
Mobile phase: 100 mM PB + 200 mM arginine hydrochloride, 5% isopropanol (pH 6.8);
Flow rate: 0.6 mL/min;
Detection wavelength: 280 nm;
Column temperature: 30 °C;
Loading volume: 10 uL;
Elution gradient: isocratic elution.

(5) Native MS:

Chromatographic column: ACQUITY UPLC Protein BEH SEC 200A (1.7 μm, 2.1 mm×150 mm);
Mobile phase: 50 mM ammonium acetate;
Running time: 12 min;
Flow rate: 0.065 mL/min;
Loading volume: 2 μL;
Column temperature: 30 °C;
Detection wavelength: 280 nm;
Capillary: 3.0 kV;
Cone voltage: 140 V;
Source offset: 80 V;
Source temperature: 125 °C;
Desolvation temperature: 250 °C;
Desolvation gas flow: 600 L/h.

(6) LC-MS:

Liquid chromatography-mass spectrometry (LC-MS) is an orthogonal technique for analyzing DAR of and drug distribution in ADCs.

Sample treatment: the sample was deglycosylated using 1 μL of PNGase F per 100 μg mAb overnight at 37 °C. Before the analysis was started, the sample was reduced through incubation with 20 mmol/L Dithiothreitol (DTT) at 37 °C for 30 min. The sample was diluted to 1 mg/mL before loading.

The chromatographic conditions for LC-MS were as follows:

Chromatographic column: PLRP-S chromatography column (Agilent), 5 μm, 1000 Å, 2.1 mm×50 mm;

Mobile phase: mobile phase A: 0.1% formic acid and 0.025% trifluoroacetic acid in water; mobile phase B: 0.1% formic acid and 0.025% trifluoroacetic acid in acetonitrile;

Flow rate: 0.25 mL/min;

The electrospray voltage was set to 4500-5000 V, the source temperature was set to 350 °C, and the curtain gas and the atomizing gas were set to 40;

Loading amount: 15-20 μg;

Chromatographic gradient used for LC-MS analysis :

| Time (min) | Mobile phase A (%) | Mobile phase B (%) |
|---|---|---|
| 0 | 10 | 90 |
| 10 | 10 | 90 |
| 30 | 90 | 10 |
| 35 | 90 | 10 |
| 36 | 10 | 90 |
| 45 | 10 | 90 |

(7) Solution replacement:

Ultrafiltration centrifugal tubes (30 KDa) were used, and solution replacement was performed by centrifugation at 6500 r/min, unless otherwise specified.

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

**Example 1** Experimental screening for antibody reduction conditions

a. Screening of the reducing agents

Pertuzumab was displaced into 50 mM sodium dihydrogen phosphate - disodium hydrogen phosphate buffer, pH 7.4 and diluted to 12.2 mg/ml. An aqueous solution of Dithiothreitol (DTT) or Tricarboxyethylphosphine (TCEP) having a concentration of 1.0 mg/ml in an amount of 8.5 molar equivalents of the antibody was added thereto, and the mixture obtained was reacted at 35 °C for 1.5 hours. Then, C-3 previously dissolved in dimethylacetamide (DMA) was added into the mixture which was then stirred at 25 °C for 1 hour in the 50 mM sodium dihydrogen phosphate - disodium hydrogen phosphate buffer for conjugation. When the conjugation completed, the solution obtained was replaced with 50 mM sodium dihydrogen phosphate - disodium hydrogen phosphate buffer, pH 7.4, which was then sampled for detection by HIC-HPLC. The results are shown in Table 1-1.

Table 1-1. Conjugation results of the antibody after reduction with different reducing agents

| Reducing agent | HIC-HPLC (D3%/D4%/D5%) |
|---|---|
| DTT | 12.93/77.93/8.24 |
| TCEP | 7.59/81.87/10.05 |

The results showed that there was no significant difference between the final results obtained using the reducing agents TCEP and DTT.

**b.** Screening of the amounts of reducing agents

Pertuzumab was displaced into 50 mM sodium dihydrogen phosphate - disodium hydrogen phosphate buffer, pH 7.4 and diluted to 12 mg/ml. Aqueous solutions of TECP and DTT in amounts of different equivalents of the antibody were added thereto, and the mixture obtained was reacted at 35 °C for 1.5 hours. The reaction systems were sampled for detection by D-SEC, and the amounts of the antibody remained unreduced therein were analyzed. The results are shown in Table 1-2.

Table 1-2. Amounts of the antibody remained unreduced after reduction with different equivalents of reducing agents

| Reducing agent | Equivalent of reducing agent | D-SEC (%) |
|---|---|---|
| TCEP | 100 | 1.2 |
| | 20 | 1.4 |
| | 10 | 1.3 |
| | 6.5 | 1.5 |
| | 5.5 | 10.7 |
| DTT | 100 | 1.3 |
| | 20 | 1.2 |
| | 10 | 1.5 |
| | 6.5 | 1.7 |
| | 5.5 | 1.8 |
| | 5.0 | 8.9 |

The results showed that the antibody was fully reduced when TCEP in an amount of ≥ 6.5 equivalents of the antibody or DTT in an amount of ≥ 5.5 equivalents of the antibody was used, and a preferred equivalent range for the reducing agents was 6.5-10.

**Example 2** Experimental screening for antibody conjugation conditions

a. Screening of the equivalents of small molecules

Pertuzumab was displaced into 50 mM sodium dihydrogen phosphate - disodium hydrogen phosphate buffer, pH 7.4, and diluted to 12 mg/ml. An aqueous solution of TECP at a concentration of 10 mg/ml was added thereto, and the mixture obtained was incubated at 35 °C for 2 hours. Afterwards, the reduced pertuzumab was displaced into 50 mM sodium dihydrogen phosphate - disodium hydrogen phosphate + 100 mM NaCl + 2 mM EDTA buffer, pH 7.4, and diluted to 8 mg/ml. Small molecule compound C-3 in the range of 4.5-6 equivalents was dissolved in organic solvent DMA and then added into the reaction system, in which the volume of DMA was made to account for 10% of the whole reaction volume. The reaction system obtained was heated and stirred for 60 min. When the reaction completed, the antibody-drug conjugates were displaced into 50 mM sodium dihydrogen phosphate - disodium hydrogen phosphate buffer, pH 7.4, using an ultrafiltration centrifugal tube, to remove the excessive compound C-3 for the conjugation reaction and reduce the content of DMA solvent in the solution. The components of the antibody-drug conjugates were analyzed by hydrophobic chromatography HIC-HPLC, and the results are shown in Table 2-1.

Table 2-1. Effects of different equivalents of the small molecule on conjugation results

| Equivalent of the small molecule | D3% | D4% | D5% |
|---|---|---|---|
| 6.5 | 5.63 | 66.02 | 26.21 |
| 6.0 | 7.82 | 73.22 | 18.81 |
| 5.5 | 6.97 | 75.63 | 17.23 |
| 5.0 | 6.67 | 78.83 | 13.91 |
| 4.5 | 15.32 | 65.25 | 6.54 |

The results showed that when the small molecule was reacted in an amount of 5.5±0.5 equivalent of the antibody, the reaction system was relatively clear, and the proportions of the main peak (D4) shown in the HIC-HPLC spectra were relatively higher.

**b.** Screening of the reaction pH:

Pertuzumab was displaced into 50 mM sodium dihydrogen phosphate - disodium hydrogen phosphate buffer, pH 7.4, and diluted to 12 mg/ml. An aqueous solution of TECP at a concentration of 10 mg/ml was added thereto, and the mixture obtained was incubated at 35 °C for 2 hours. Afterwards, the reduced pertuzumab was displaced into 50 mM sodium dihydrogen phosphate - disodium hydrogen phosphate + 100 mM NaCl + 2 mM EDTA buffer, at a pH value in the range of 6.8-7.8, and diluted to 5 mg/ml. Small molecule compound C-3 in an amount of 5.0 equivalents was dissolved in organic solvent DMA and then added into the reaction system in which the volume of DMA was made to account for 10% of the whole reaction volume. The reaction system obtained was heated and stirred for 60 min. When the reaction completed, the antibody-drug conjugates were displaced into 50 mM sodium dihydrogen phosphate - disodium hydrogen phosphate buffer, pH 7.4. The components of the antibody-drug conjugates were analyzed by hydrophobic chromatography HIC-HPLC, and NR-CE-SDS and the results are shown in Table 2-2.

Table 2-2. Effect of different pH on conjugation results

| pH | HIC-HPLC (D3%/D4%/D5%) | Purity by NR-CE-SDS (%) |
|---|---|---|
| 6.5 | 7.57/78.76/13.49 | 93.8 |
| 6.7 | 5.89/83.12/10.98 | 94.8 |
| 7.0 | 5.62/81.82/12.56 | 94.7 |
| 7.3 | 5.38/80.72/13.91 | 94.3 |
| 7.6 | 3.88/82.47/10.65 | 93.9 |
| 7.8 | 8.12/76.53/15.17 | 90.3 |

The results showed that when a pH value in the range of 6.5-7.6 was used in the reaction, the reaction system was relatively clear, the proportions of the main peak (D4) shown in the HIC-HPLC spectra were relatively higher, and the purity determined by NR-CE-SDS was good.

c. Screening of the reaction co-solvents and percentages they account for

Pertuzumab was reduced as described in section b in this Example. When the reduction completed, the antibody was displaced, diluted to about 5.0 mg/ml, and samples of the diluted antibody solution obtained were grouped. As shown in Table 2-3, compound C-3 was dissolved to 20 mg/mL in one of organic solvents N,N-Dimethylacetamide (DMA), Dimethylsulfoxide (DMSO), and Acetonitrile (ACN) in different groups. Then, volumes of the compound C-3 solutions needed to be added into the reaction systems of C-3 and the antibody were calculated according to the amount of C-3 which was 5 molar equivalents of the antibody, and the respective solvents were supplemented to the reaction systems of the two to achieve the volume percentages (based on the final reaction systems) as shown in Table 2-3. The reaction systems were stirred at 25 °C for 1 hour. Then the antibody-drug conjugates were displaced into 50 mM sodium dihydrogen phosphate - disodium hydrogen phosphate buffer, pH 7.4, and sampled respectively. The components of the antibody-drug conjugates were analyzed by hydrophobic chromatography HIC-HPLC and NR-CE-SDS and the results are shown in Table 2-3.

Table 2-3. Conjugation results when different organic solvents were used as co-solvents

| Name of solvent | Volume of solvent (%) | Appearance of reaction system | HIC-HPLC (D3%/D4%/D5%) | NR-CE-SDS (%) |
|---|---|---|---|---|
| DMA | 40 | turbid | 3.94/77.15/19.38 | 76.9 |
| | 30 | clear | 3.96/79.78/16.37 | 87.6 |
| | 20 | clear | 4.41/80.09/15.37 | 88.9 |
| | 15 | clear | 6.37/80.12/13.45 | 89.3 |
| | 10 | clear | 7.59/81.87/10.05 | 94.4 |
| | 8 | clear | 7.43/82.65/9.54 | 94.6 |
| | 6 | clear | 7.59/81.87/10.05 | 93.7 |
| | 4 | clear | 12.54/76.87/9.56 | 92.1 |
| DMSO | 40 | clear | 3.54/75.15/21.28 | 76.6 |
| | 30 | clear | 3.85/79.73/16.41 | 86.3 |
| | 20 | clear | 4.22/80.05/15.64 | 87.9 |
| | 15 | clear | 6.34/80.02/13.54 | 89.1 |
| | 10 | clear | 12.93/77.93/8.24 | 93.9 |
| | 8 | clear | 10.34/79.45/8.16 | 94.1 |
| | 6 | clear | 10.27/78.73/7.98 | 92.9 |
| | 4 | turbid | 20.10/70.65/7.92 | 90.6 |
| ACN | 40 | turbid | 4.97/73.65/21.17 | 77.4 |
| | 30 | clear | 4.46/78.63/16.72 | 87.1 |
| | 20 | clear | 5.23/79.89/14.76 | 88.6 |
| | 15 | clear | 6.46/80.02/13.47 | 89.7 |
| | 10 | clear | 11.12/73.93/12.24 | 93.4 |
| | 8 | clear | 10.13/75.93/11.82 | 95.3 |
| | 6 | clear | 10.13/75.93/11.82 | 93.6 |
| | 4 | turbid | 17.54/68.91/12.13 | 90.72 |

The results showed that DMA, DMSO and ACN all could be used as co-solvents for this step of the process, but DMA and DMSO were preferred. The conjugation reaction systems were clear and the respective reaction results were good when the volume of co-solvent DMA accounted for more than 4% and no more than 30% of the reaction system volume, or the volumes of co-solvents DMSO and ACN accounted for more than 6% and no more than 30% of the reaction system volume.

**d.** Screening of the antibody concentrations:

Pertuzumab was reduced as described in section a in this Example. When the reduction completed, the antibody was displaced into 50 mM sodium dihydrogen phosphate - disodium hydrogen phosphate + 100 mM NaCl + 2 mM EDTA buffer, pH 7.4, and diluted to a concentration in the range of 2.5-13 mg/ml. A solution of Compound C-3 at 20 mg/ml was formulated in DMA, and was added into the reaction system in an amount of 5 equivalents of the antibody, and then the reaction system was stirred at 25 °C for 1 hour. When the conjugation completed, the solution system was replaced with 50 mM sodium dihydrogen phosphate - disodium hydrogen phosphate buffer, pH 7.4, the components of the antibody-drug conjugates were analyzed by hydrophobic chromatography HIC-HPLC and NR-CE-SDS, and the results are shown in Table 2-4.

Table 2-4. Effects of antibody concentrations on conjugation results

| Antibody concentration (mg/ml) | HIC-HPLC (D3%/D4%/D5%) | NR-CE-SDS (%) |
|---|---|---|
| 2.5 | 15.21/74.68/8.99 | 89.4 |
| 3.5 | 11.58/80.12/8.19 | 91.7 |
| 5.0 | 11.32/81.23/7.36 | 91.1 |
| 7 | 10.54/83.72/5.67 | 90.6 |
| 10 | 11.31/81.47/7.56 | 90.5 |
| 13 | 14.31/75.68/9.56 | 88.5 |

As shown in Table 2-4, good conjugation results were obtained at antibody concentrations of 3.5-10 mg/ml. Therefore, the antibody concentration for the antibody-drug conjugates reaction can be selected to be 3.5-10 mg/ml.

e. Screening of the reaction temperatures:

Pertuzumab was reduced as described in section a in this Example. When the reduction completed, the antibody was displaced into 50 mM sodium dihydrogen phosphate - disodium hydrogen phosphate + 100 mM NaCl + 2 mM EDTA buffer, pH 7.4, and diluted to a concentration of about 5 mg/ml. The reaction system was pre-warmed to 25±10°C, followed by the addition of a solution of compound C-1 in DMA. Then the reaction system was stirred at respective temperature shown below for 1 hour. When the reaction completed, the solution system was replaced with 50 mM sodium dihydrogen phosphate - disodium hydrogen phosphate buffer, pH 7.4, the components of the antibody-drug conjugates were analyzed by hydrophobic chromatography HIC-HPLC and NR-CE-SDS, and the results are shown in Table 2-5.

Table 2-5. Effects of different reaction temperatures on conjugation results

| Reaction temperature (°C) | HIC-HPLC (D3%/D4%/D5%) | SEC main peak proportion (%) | Purity by NR-CE-SDS (%) |
|---|---|---|---|
| 15 | 6.77/81.93/11.30 | 98.0 | 95.3 |
| 20 | 7.13/81.63/11.24 | 97.9 | 95.9 |
| 25 | 9.37/80.80/9.41 | 97.7 | 95.0 |
| 30 | 8.05/80.84/10.77 | 97.2 | 94.9 |
| 35 | 8.16/79.94/10.76 | 97.3 | 95.1 |

The results showed that the reaction results were all good at a temperature in the range of 25±10°C. Thus, 25±10°C is a selectable temperature range.

**Example 3** Experimental screening for antibody hydrolysis conditions

a. Screening of reaction temperatures, pH and time:

Pertuzumab was reduced and conjugated as described in section a in Example 2. When the conjugation completed, the antibody-drug conjugates were displaced into 50 mM sodium dihydrogen phosphate - disodium hydrogen phosphate buffer, at a pH value in the range of 7.4-9.0. The reaction solutions were stirred at a temperature in the range of 25-45 °C for 3 h, and observed for color change. The results are shown in Table 3-1.

Table 3-1. Hydrolysis of antibody-drug conjugates at different temperatures and pH

| Temperature (°C) | pH | Time period of hydrolysis | Color | Results of hydrolysis |
|---|---|---|---|---|
| 25 | 7.4 | 6h | light yellow | partially hydrolyzed |
| 30 | 7.4 | 4 h | light yellow | partially hydrolyzed |
| 25 | 7.6 | 4h | light yellow | partially hydrolyzed |

(continued)

| Temperature (°C) | pH | Time period of hydrolysis | Color | Results of hydrolysis |
|---|---|---|---|---|
| 30 | 7.6 | 4h | light yellow | partially hydrolyzed |
| 35 | 7.6 | 4h | light yellow | partially hydrolyzed |
| 30 | 7.8 | 3h | light yellow | partially hydrolyzed |
| 35 | 7.8 | 2h | substantially colorless and transparent | totally hydrolyzed |
| 30 | 8.0 | 6h | colorless and transparent | totally hydrolyzed |
| 35 | 8.0 | 4h | colorless and transparent | totally hydrolyzed |
| 45 | 8.5 | 2h | colorless and transparent | totally hydrolyzed |
| 45 | 9.0 | 2h | colorless and transparent | antibody deamidated apparently |

The results showed that the antibody-drug conjugates could be hydrolyzed smoothly at a temperature of 35±10 °C and pH 8.0±0.5 with a hydrolysis time ≥2 hours.

**b.** Screening of small molecules:

Pertuzumab was reduced and conjugated as described in section a in Example 2. Conjugated samples obtained using different small molecule compounds were subjected to hydrolysis at 35 °C for a time period in the range of 3-18 hours, in 50 mM sodium dihydrogen phosphate - disodium hydrogen phosphate buffer, pH 7.8. The hydrolysis systems were sampled at different time points, and detected by Native MS. The percentages of hydrolyzed ADCs in the samples were detected, and the results are shown in Table 3-2.

Table 3-2. Results detected by Native MS of the conjugated samples obtained using different small molecule compounds subjected to hydrolysis

| Small molecule | DAR (LC-MS) | Molecular weight of the naked antibody measured | Molecular weight of ADCs before hydrolysis | Molecular weight of ADCs subjected to hydrolysis for 3 h | Molecular weight of ADCs subjected to hydrolysis for 6 h | Molecular weight of ADCs subjected to hydrolysis for 18 h | 3 h hydrolysis percentage (%) | 6 h hydrolysis percentage (%) | 18 h hydrolysis percentage (%) |
|---|---|---|---|---|---|---|---|---|---|
| **C-3** | 3.99 | 148089.44 | 154181.23 | 154235.9 | 154236.8 | NA | 98.31 | 99.56 | NA |
| **C-1** | 3.99 | 148085.99 | 154032.2 | 154078.14 | 154091.94 | NA | 79.08 | 98.25 | NA |
| **A-2** | 3.99 | 148087.56 | 154268.54 | 154282.13 | 154291.12 | 154304.15 | 17.85 | 30.33 | 48.43 |
| **A-4** | 3.98 | 148087.56 | 154293.16 | 154311.87 | 154320.87 | 154331.65 | 25.99 | 38.49 | 53.46 |
| **A-5** | 3.98 | 148087.92 | 154025.21 | 154030.45 | 154037.27 | 154037.94 | 7.29 | 16.76 | 28.81 |
| **B-6** | 3.99 | 148088.12 | 154293.16 | 154309.62 | 154317.15 | 154327.43 | 22.86 | 33.32 | 47.60 |
| **B-7** | 3.99 | 148086.67 | 154140.12 | 154165.79 | 154180.63 | 154196.07 | 34.10 | 54.71 | 76.15 |
| **D-1** | 3.97 | 148086.53 | 153354.23 | 153360.45 | 153362.56 | 153372.48 | 4.74 | 7.67 | 21.44 |

The results showed that when the small molecule compounds C-1 and C-3 were used, the conjugated samples were hydrolyzed all most completely within 3 hours or 6 hours; and the conjugated samples obtained using A-2, A-4, B-6 and B-7 could be hydrolyzed smoothly in the buffer at 35 °C, but with a relatively low percentage of hydrolysis.

**c.** Screening of hydrolysis systems:

Pertuzumab was reduced and conjugated as described in section a in Example 2. Conjugated samples obtained using different small molecule compounds were displaced to different hydrolysis solution systems (A: 50 mM sodium dihydrogen phosphate - disodium hydrogen phosphate buffer, pH 7.8; B: 50 mM sodium dihydrogen phosphate - disodium hydrogen phosphate buffer + 3% arginine, pH 7.8; C: 50 mM potassium dihydrogen phosphate - dipotassium hydrogen phosphate buffer, pH 7.8; and D: 50 mM tromethamine - hydrochloric acid buffer, pH 7.8) for hydrolysis. Then the hydrolysis systems were sampled at different time points, and detected by Native MS. The percentages of hydrolyzed ADCs in the samples were detected, and the results are shown in Table 3-3.

Table 3-3. Hydrolysis percentages in different hydrolysis systems

| Small molecule | A | | B | | C | | D | |
|---|---|---|---|---|---|---|---|---|
| | 1 h hydrolysis percentage (%) | 3 h hydrolysis percentage (%) | 1 h hydrolysis percentage (%) | 3 h hydrolysis percentage (%) | 1 h hydrolysis percentage (%) | 3 h hydrolysis percentage (%) | 1 h hydrolysis percentage (%) | 3 h hydrolysis percentage (%) |
| **C-3** | 90.31 | 99.56 | 97.52 | 99.41 | 90.55 | 99.70 | 88.36 | 99.34 |
| **C-1** | 85.31 | 99.56 | 94.52 | 99.87 | 87.52 | 99.70 | 84.78 | 99.79 |
| **A-3** | 89.31 | 99.32 | 93.48 | 99.41 | 92.52 | 99.70 | 90.33 | 99.31 |
| **A-4** | 15.65 | 25.99 | 18.17 | 35.23 | 14.98 | 26.13 | 14.56 | 25.32 |
| **B-7** | 15.83 | 34.10 | 26.12 | 51.25 | 15.12 | 35.12 | 16.62 | 36.43 |

The results showed that when the small molecule compounds C-3, C-1 and A-3 were used, the conjugated samples were hydrolyzed rapidly in all above buffers, with a faster hydrolysis speed in the buffer containing Arginine. While the conjugated samples obtained using compounds A-4 and B-7 required more time for being hydrolyzed at the same pH values.

**d.** Screening of ADC concentrations

Pertuzumab was reduced and conjugated as described in section a in Example 2. Conjugated samples obtained were displaced to a hydrolysis system of 50 mM sodium dihydrogen phosphate - disodium hydrogen phosphate buffer, pH 7.8, to achieve different concentrations. The hydrolysis systems were sampled at different time points, and detected by Native MS. The percentages of hydrolyzed ADCs in the samples were detected, and the results are shown in Table 3-4.

Table 3-4. Hydrolysis speeds of antibody-drug conjugates at different concentrations

| Concentration (mg/ml) | 1 h hydrolysis percentage (%) | 2 h hydrolysis percentage (%) | 3 h hydrolysis percentage (%) |
|---|---|---|---|
| 2 | 74.53 | 86.63 | 89.51 |
| 5 | 87.14 | 97.25 | 98.22 |
| 10 | 97.43 | 98.62 | 99.07 |
| 12 | 98.01 | 99.19 | 99.63 |
| 15 | 98.05 | 99.23 | 99.58 |
| 20 | 93.26 | 99.27 | 99.22 |
| 30 | 75.93 | 79.82 | 83.12 |

The results showed that when the concentration of ADCs was less than 5 mg/mL or more than 20 mg/mL, the hydrolysis percentages were still less than those when the concentration of ADCs was in the range of 5-20 mg/mL, even the hydrolysis reaction time was prolonged.

**Example 4** Experimental screening for antibody purification conditions

Conductivity adjustment was performed on the hydrolyzed antibody-drug conjugates using high-concentration ammonium sulfate. Conjugated samples were filtered and loaded onto chromatographic columns with different fillers shown below, after their conductivity was adjusted to be close to the conductivity value of the respective start buffer. The loaded samples were eluted using the start buffer, and finally the purified samples were pooled and collected.

**a.** Screening of the fillers:

Pertuzumab was reduced and conjugated as described in section a in Example 2. Conjugated samples obtained were displaced to 50 mM sodium dihydrogen phosphate - disodium hydrogen phosphate buffer, pH 7.8 and incubated for longer than 3 hours at 35 °C. The obtained antibody-drug conjugates were used for filler screening. Column chromatography was performed on the fillers of different ligands and different supports under the same chromatography condition, and the types of the screened fillers and the screening results are shown in Table 4-1.

Table 4-1. Screening results of different types of fillers

| Filler | Yield (%) | Results |
|---|---|---|
| Butyl Sepharose 4FF | 70 | Most of D3 flowed through, and a little of D4 flowed through |
| Butyl Sepharose HP | 65 | D3 was substantially separated from D4, but D4 could not be eluted completely |
| Capto Butyl Impres | 65 | D3 was poorly separated from D4 |
| Capto Phenyl Impres | 68 | D3 was substantially separated from D4, but D4 could not be eluted normally |
| Capto Butyl | 70 | D3 was poorly separated from D4 |
| Butyl 650M | NA | Strongly bounded and could not be eluted normally |

The results showed that Butyl Sepharose 4FF, Butyl Sepharose HP, and Capto Phenyl Impres could be used to purify the conjugated sample. Thus, the conjugated sample can be purified using Butyl Sepharose 4FF and by a protocol enabling the flowing through of D3, taking into account the yield and sample properties.

**b.** Screening of salts required for chromatography and their concentrations:

Pertuzumab was reduced and conjugated as described in section a in Example 2. Conjugated samples obtained were displaced to 50 mM sodium dihydrogen phosphate - disodium hydrogen phosphate buffer, pH 7.8 and diluted to 12 mg/mL. When hydrolysis at 35 °C for 3 hours completed, solutions of 50 mM sodium dihydrogen phosphate - disodium hydrogen phosphate containing sodium chloride at a concentration in the range of 1.0-2.0 mol/L (as the salt required for chromatography), or solutions of 50 mM sodium dihydrogen phosphate - disodium hydrogen phosphate containing ammonium sulfate at a concentration in the range of 0.325-0.45 mol/L (as the salt required for chromatography), were used as the buffers for the stationary phase; and a solution of 50 mM sodium dihydrogen phosphate - disodium hydrogen phosphate was used as the eluting solution, and column chromatography using Butyl Sepharose 4FF was performed on the conjugated samples of pertuzumab to a compound subjected to hydrolysis. The results of separation were shown in Table 4-2.

Table 4-2. Effects of different salts and salt concentrations on the purification results

| Salt in the chromatography buffer for the stationary phase | Concentration of the salt in the chromatography buffer for the stationary phase (mol/L) | Loading amount (mg/ml) | Separation effect | Yield (%) | HIC-HPLC main peak (D4) proportion (%) |
|---|---|---|---|---|---|
| Sodium chloride | 1.0 | 10 | the sample flowed through | NA | NA |
| | 1.5 | 10 | good | 70 | 95 |
| | 1.5 | 15 | good | 67 | 96 |
| | 1.5 | 20 | the sample flowed through | NA | NA |
| | 2.0 | 20 | good | 65 | 94 |
| Ammonium sulfate | 0.325 | 10 | good | 75 | 95 |
| | 0.325 | 15 | the sample flowed through | NA | NA |
| | 0.45 | 15 | good | 76 | 90 |
| | 0.45 | 20 | good | 75 | 96 |

The results showed that both sodium chloride and ammonium sulfate could be used in the column chromatography process, and 0.45 mol/L ammonium sulfate was preferred as the salt in the chromatography buffer for the stationary phase, taking into account the yield and sample heterogeneity.

**Example 5** Experimental screening for scaled-up process conditions

The process was scaled up to ≥100 mg of the antibody and evaluated for its scalability, and the conjugation conditions in the process were further adjusted.

Pertuzumab in an amount of ≥100 mg was displaced into 50 mM sodium dihydrogen phosphate - disodium hydrogen phosphate buffer, pH 7.4 and diluted to a protein concentration of 12 mg/ml. An aqueous solution of TECP at 10 mg/ml (TCEP was in an amount of 6.5 equivalents of the antibody) was added to the buffer, and the obtained reaction system was incubated at 35 °C for 2 hours. Then, the reaction system was replaced with 50 mM sodium dihydrogen phosphate - disodium hydrogen phosphate + 100 mM NaCl + 2 mM EDTA buffer, pH 7.2, and the antibody was diluted to 8 mg/ml. Small molecule compound C-3 in an amount of equivalents shown in the Table below and previously dissolved in DMA was then added into the buffer, followed by the further addition of DMA to make the volume of DMA account for

10% of the whole reaction volume. The reaction system obtained was heated and stirred for 1 hour. When the conjugation completed, the reaction system was replaced with 50 mM sodium dihydrogen phosphate - disodium hydrogen phosphate buffer, pH 7.8, in which the concentration of the antibody-drug conjugates were diluted to 12 mg/mL. The solution of the antibody-drug conjugates obtained was heated at 35 °C for 3 hours, and detected by HIC-HPLC. Results were shown in Table 5-1.

Table 5-1. Experimental results of reaction scales ≥100 mg under different reaction conditions

| Reaction scale | Equivalent of the small molecule | Means for solution replacement | HIC-HPLC main peak (D4) proportion (main peak area before purification) (%) | Hydrolysis percentage (%) |
|---|---|---|---|---|
| 100 mg | 4.8 | centrifugal filtration | 77.3 | 100 |
| 126 mg | 4.8 | Gel chromatography (G25) | 79.5 | 100 |
| 152 mg | 4.5 | Gel chromatography (G25) | 81.2 | 100 |
| 157 mg | 4.8 | Gel chromatography (G25) | 82.3 | 100 |
| 149 mg | 4.5 | Gel chromatography (G25) | 80.7 | 100 |
| 154 mg | 5.0 | Gel chromatography (G25) | 83.6 | 100 |
| 198 mg | 4.8 | Gel chromatography (G25) | 82.1 | 100 |
| 204 mg | 4.8 | Gel chromatography (G25) | 80.1 | 100 |
| 307 mg | 4.8 | Gel chromatography (G25) | 79.8 | 100 |
| 294 mg | 4.8 | Gel chromatography (G25) | 77.5 | 100 |
| 3.5 g | 4.5 | UF/DF | 83.2 | 100 |
| 3.2 g | 4.8 | UF/DF | 82.7 | 100 |

The results showed that with a scale ≥100mg, the method according to the present invention could obtain conjugation results substantially the same as those obtained by a laboratory process, and the conjugation process could be smoothly scaled-up.

**Example 6** Conjugation experiments of different small molecule compounds to antibody

Pertuzumab was displaced into 50 mM sodium dihydrogen phosphate - disodium hydrogen phosphate buffer, pH 7.4, and diluted to a protein concentration of about 12 mg/ml. An aqueous solution of TECP at 10 mg/ml (TCEP was in an amount of 6.5 equivalents of the antibody) was added to the buffer, and the obtained reaction system was incubated

at 35 °C for 2 hours. Then, the reaction system was replaced with 50 mM sodium dihydrogen phosphate - disodium hydrogen phosphate + 100 mM NaCl + 2 mM EDTA buffer, pH 7.2, and the antibody was diluted to 8 mg/ml. Small molecule compound A-1 or C-3 (0.6 eq) dissolved in DMA which was also used as the co-solvent was then added into the buffer, and the reaction system obtained was heated and stirred for 1 hour. Then through centrifugal ultrafiltration the reaction system was replaced with 50 mM sodium dihydrogen phosphate - disodium hydrogen phosphate buffer, pH 7.8, in which the concentration of the antibody-drug conjugates were diluted to 12 mg/mL. The solution of the antibody-drug conjugates obtained was incubated at 35 °C for 3 hours. When the hydrolysis completed, with conductivity adjusted using 3 M ammonium sulfate, the solution was purified through chromatography. For performing the chromatography, Butyl Sepharose 4FF filler, 50 mM sodium dihydrogen phosphate - disodium hydrogen phosphate buffer (containing 0.45 M ammonium sulfate) (i.e., the start buffer), and 50 mM sodium dihydrogen phosphate - disodium hydrogen phosphate as the eluent were used, with the pH values of both the start buffer and the eluent were set to 7.5. The resulting purified solution was concentrated and replaced with 50 mM sodium dihydrogen phosphate - disodium hydrogen phosphate buffer, pH 7.4, and then was detected by HIC-HPLC, SEC-HPLC, and NR-CE-SDS respectively. The results are shown in Table 6-1.

Table 6-1. Results of conjugation experiments of different small molecule compounds to antibody

| Compound | Antibody | Conjugation scale | Yield (%) | HIC-HPLC main peak (D4) proportion (%) | SEC-HPLC Aggregates (%) | NR-CE-SDS (%) |
|---|---|---|---|---|---|---|
| A-1 | Pertuzumab | 150 mg | 70 | 97 | 1.3 | 97.7 |
| C-3 | Pertuzumab | 120 mg | 67 | 97 | 1.2 | 96.4 |

As can be seen from the data in the Table, compounds A-1 and C-3 both were suitable for the conjugation process, and the results were well consistent.

**Example 7** Large-scale conjugation experiments of different small molecule compounds to antibodies

IgG1 antibodies against different targets including HER2 (Pertuzumab) and CD20 (Rituximab) were conjugated to small molecule compounds according to the process as described in Example 6, and then the prepared antibody-drug conjugates were detected. The results showed the process had good applicability to IgG1 antibodies against different targets. The results are shown in Table 7-1 and Figure 1.

Table 7-1. Results of large-Scale conjugation experiments of different small molecule compounds to antibodies

| Antibody | Small molecule compound | Conjugation scale | HIC-HPLC main peak (D4) proportion (in the final product after purification) (%) | SEC-HPLC Aggregates (%) | NR-CE-SDS (%) |
|---|---|---|---|---|---|
| Pertuzumab | C-3 | 3.2 g | 96.40 | 0.6 | 95.6 |
| Rituximab | C-3 | 10 g | 96.88 | 1.35 | 96.08 |

It can be seen that the IgG1 antibodies having different antibody sequences against different targets, are all suitable for the ADC preparation method provided by the present disclosure.

**Example 8** Physicochemical properties, in vitro/in vivo stability, in vivo efficacy and safety of ADCs prepared by different processes

According to the process as described in Example 6, 2.5 g of Pertuzumab was reduced and conjugated to the small molecule compound C-1 in 50 mM sodium dihydrogen phosphate - disodium hydrogen phosphate + 100 mM NaCl + 2 mM EDTA buffer, pH 7.2. The antibody-drug conjugates obtained were displaced in a buffer (pH 7.8) using UF/DF, and the final product obtained was sampled and grouped into group A and group B.

The sample in group A was directly purified by Butyl Sepharose 4FF filler, and the resulting purified solution was concentrated and replaced with 50 mM sodium dihydrogen phosphate - disodium hydrogen phosphate buffer, pH 7.4, to obtain a solution having a concentration of 10 mg/mL (solution A). The sample in group B was incubated at 35 °C for 3 hours and then like the sample in group A, was purified by Butyl Sepharose 4FF filler and the resulting purified solution was concentrated and replaced with 50 mM sodium dihydrogen phosphate - disodium hydrogen phosphate buffer, pH 7.4, to obtain a solution having a concentration of 10 mg/mL (solution B).

**a.** Evaluation of physicochemical properties:

The solution A and solution B were sampled and subjected to analysis by mass spectrometry, non-reducing electrophoresis (NR-CE-SDS), hydrophobic chromatography, and SEC-HPLC, respectively. The results showed that although the purities measured by HIC-HPLC and SEC-HPLC of both solution A and solution B increased due to the purification processing, solution B had a higher purity measured by NR-CE-SDS. The results are shown in Table 8-1 and Figure. 2.

Table 8-1. Analysis results of physicochemical properties of conjugated samples obtained by different processes

| Group | Molecular weight measured by LC-MS | HIC-HPLC (%) | SEC-HPLC aggregates (%) | NR-CE-SDS (%) |
|---|---|---|---|---|
| A | 154068 | 93.9 | 0.6 | 93.9 |
| B | 154088 | 93.8 | 0.7 | 98.2 |

**b.** Evaluation of in vivo stability in plasma:

Solution A and solution B were sampled and diluted to 1 mg/ml in physiological saline and then administered to cynomolgus monkeys at a dose of 6 mg/kg. Blood was collected at 30 min, 1 h, 2 h, 4 h, 12 h, 24 h, 48 h, 72 h and 128 h after administration, respectively. Calibrated MMAE (MCE: HY-15162) was used as the standard, to detect free MMAE in the plasma samples. The results showed that while the maximum peak of free MMAE detected for both solution A and solution B appeared at 24 h, the exposed amounts of free MMAE in the plasma samples from the animals administered with solution B were significantly lower than those in the plasma samples from the animals administered with solution A, indicating that the conjugated sample in group B which were subjected to hydrolysis were more stable in vivo. The results are shown in Table 8-2.

Table 8-2. Analysis results of free MMAE of conjugated samples obtained by different processes

| Solutions administered | Gender | Animal ID | MMAE | | | |
|---|---|---|---|---|---|---|
| | | | t1/2 | Tmax | Cmax | AUClast |
| | | | (h) | (h) | (ng/mL) | (h-ng/mL) |
| A | Male | 20-3301 | 48.3 | 24 | 0.46 | 41.71 |
| | Female | 20-3302 | 52.6 | 24 | 0.48 | 56.27 |
| B | Male | 20-3303 | 48 | 24 | 0.24 | 11.95 |
| | Female | 20-3304 | 19.09 | 24 | 0.4 | 14.77 |

**c.** Detection of biochemical indexes in vivo

2.5-year old or older cynomolgus monkeys were grouped randomly into 2 groups, each including one male and one female cynomolgus monkeys. Solution A and solution B were sampled and diluted to 1 mg/ml in physiological saline and then administered to the cynomolgus monkeys by intravenous dripping at a dose of 6 mg/kg. Seven days after administration, blood biochemical indexes of the monkeys were analyzed. The results showed that much more abnormal indexes were detected from solution A than from solution B, to a more severe extent.

Table 8-3. Analysis results of in vivo efficacy of conjugated samples obtained by different processes

| Index | Group A | | Group B | |
|---|---|---|---|---|
| | Male | Female | Male | Female |
| WBC | 2.18 | 1.5 | 4.16 | 9.66 |
| ($10^9$/L) | (↓55%) | (↓68%) | (↓14%) | |
| Neut | 2.8 | 4.6 | 6 | 52.4 |
| (%) | (↓71%) | (↓80%) | (↓38%) | |

(continued)

<table>
<tr><td rowspan="2">Index</td><td colspan="2">Group A</td><td colspan="2">Group B</td></tr>
<tr><td>Male</td><td>Female</td><td>Male</td><td>Female</td></tr>
<tr><td>Lymph</td><td>91.8</td><td>93.2</td><td>86.8</td><td rowspan="2">39.1</td></tr>
<tr><td>(%)</td><td>(↑11%)</td><td>(↑36%)</td><td>(↑5%)</td></tr>
<tr><td>Mono</td><td rowspan="2">1.2</td><td>0.4</td><td rowspan="2">2.6</td><td rowspan="2">3.1</td></tr>
<tr><td>(%)</td><td>(↓55%)</td></tr>
<tr><td>Retic</td><td>0.2</td><td>0.09</td><td rowspan="2">0.53</td><td rowspan="2">1.7</td></tr>
<tr><td>(%)</td><td>(↓48%)</td><td>(↓77%)</td></tr>
<tr><td>PLT</td><td rowspan="2">408</td><td rowspan="2">350</td><td rowspan="2">395</td><td>650</td></tr>
<tr><td>($10^9$/L)</td><td>(↑18%)</td></tr>
<tr><td>HGB</td><td rowspan="2">130</td><td>104</td><td>107</td><td rowspan="2">122</td></tr>
<tr><td>(g/L)</td><td>(↓7%)</td><td>(↓6%)</td></tr>
<tr><td>EOS</td><td rowspan="2">0.9</td><td rowspan="2">0.6</td><td rowspan="2">2.2</td><td rowspan="2">3.5</td></tr>
<tr><td>(%)</td></tr>
<tr><td>AST</td><td rowspan="2">48</td><td>116</td><td rowspan="2">55</td><td rowspan="2">70</td></tr>
<tr><td>( U/L )</td><td>(↑14%)</td></tr>
<tr><td>ALT</td><td>99</td><td>221</td><td>59</td><td>236</td></tr>
<tr><td>(U/L)</td><td>(↑9%)</td><td>(↑135%)</td><td></td><td>(↑151%)</td></tr>
<tr><td>CK</td><td rowspan="2">295</td><td>800</td><td rowspan="2">371</td><td rowspan="2">593</td></tr>
<tr><td>(U/L)</td><td>(↑42%)</td></tr>
</table>

The above description of the embodiments of the present disclosure is not intended to limit the present disclosure, and those skilled in the art may make various changes and modifications to the present disclosure without departing from the spirit of the present disclosure, which should fall within the scope of the appended claims.

## Claims

1. A preparation method for a disubstituted bridged antibody-drug conjugate, comprising:

subjecting an antibody conjugation product obtained by conjugating an antibody to a compound represented by formula I to hydrolysis:

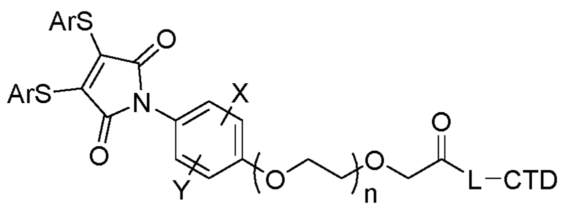

I

in formula I, Ar is phenyl or substituted phenyl, and the substituent in the substituted phenyl is selected from the group consisting of alkyl, alkoxy, halogen, ester, cyano and $-C(O)NR_1R_2-$, in which $R_1$ and $R_2$ are independently selected from the group consisting of a chemical bond, H and alkyl, or $R_1$ and $R_2$ form a 5-7 membered heterocyclic ring with one or more heteroatoms selected from the group consisting of O, N and S;

X and Y are independently hydrogen, halogen, trifluoromethyl or alkoxy;
n is any integer between 1 and 24;
L-CTD is a cytotoxic drug and a connection release structure applied in an antibody-drug conjugate.

**2.** The preparation method according to claim 1, wherein the alkyl is $C_1$-$C_6$ alkyl, preferably $C_1$-$C_3$ alkyl, more preferably methyl;

preferably, the alkoxy is $C_1$-$C_6$ alkoxy, preferably $C_1$-$C_3$ alkoxy, more preferably methoxy;
preferably, L is selected from the group consisting of Val-Ala-PAB (VA-PAB), Val-Cit-PAB (VC-PAB), Phe-Lys-PAB, and MAC glucuronide phenol linker; and CTD is, for example, a cytotoxic drug, preferably a tubulin inhibitor (e.g., tubulin inhibitors MMAE, DM1, DM4, Tublysin); a topoisomerase inhibitor (e.g., topoisomerase inhibitors SN38, Exatecan and derivatives thereof) or a DNA binding agent (e.g., DNA binding agent PBD and derivatives thereof).

**3.** The preparation method according to claim 1 or 2, wherein in formula I:

$R_1$ and $R_2$ are independently selected from the group consisting of H and $C_1$-$C_3$ alkyl; or $R_1$ and $R_2$ form a 6-membered heterocyclic ring with one or more heteroatoms selected from the group consisting of O and N, preferably morpholine;
preferably, Ar is phenyl, 4-methylformamido-substituted phenyl

( 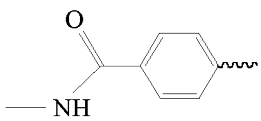 ),

or 4-formylmorpholine-substituted phenyl

( 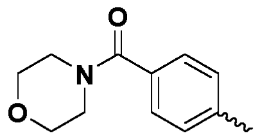 );

preferably, X and Y are independently hydrogen, fluoro, trifluoromethyl or methoxy; more preferably, X and Y are independently hydrogen, or X and Y are independently at the meta position on the phenyl ring relative to the maleimide;
preferably, n is any integer between 1 and 10, preferably between 3 and 5;
preferably, L-CTD is VC-PAB-MMAE or VC-seco-DUBA.

**4.** The preparation method according to any one of claims 1 to 3, wherein the hydrolysis is performed before or after purification of the antibody conjugation product;

preferably, the hydrolysis comprises: heating the antibody conjugation product in a hydrolysis buffer at pH 7.4-9.0 at 25-45 °C for 1-24 hours, wherein the hydrolysis buffer comprises one or more selected from the group consisting of sodium dihydrogen phosphate, disodium hydrogen phosphate, dipotassium hydrogen phosphate, potassium dihydrogen phosphate, citric acid, glycine, tromethamine, arginine hydrochloride, hydrochloric acid, phosphoric acid, sodium hydroxide, and potassium hydroxide;
preferably, the hydrolysis buffer is a sodium dihydrogen phosphate - disodium hydrogen phosphate buffer, a potassium dihydrogen phosphate - dipotassium hydrogen phosphate buffer, or a tromethamine buffer;
preferably, the concentration of the antibody conjugation product in the hydrolysis buffer is 2-30 mg/mL, preferably 5-20 mg/mL.

**5.** The preparation method according to any one of claims 1 to 4, wherein the hydrolysis comprises: heating the antibody conjugation product in the hydrolysis buffer at 25-35 °C for 1-6 hours, preferably 1-3 hours;

preferably, the hydrolysis buffer is at pH 7.5-8.5, preferably pH 7.8;

preferably, the antibody is an IgG antibody, preferably IgG1 antibody.

6. The preparation method according to any one of claims 1 to 5, wherein the preparation method comprises the following steps:

a. Antibody reduction: adding a reducing agent in an amount of ≥ 5.5 molar equivalents of the antibody to a buffer containing the antibody in a concentration of 5-30 mg/mL, and reacting the reducing agent and the antibody at 30-40 °C for 1.5-2 hours, wherein the reducing agent is one or more selected from the group consisting of TCEP, DTT, 2-MEA, and DTBA;
b. Antibody conjugation: displacing the reduced antibody obtained in step a to a phosphate buffer at pH 6.5-7.6 in which the antibody is diluted to a concentration of 3.5-15 mg/mL to obtain a diluted antibody solution; adding a compound represented by formula I in an amount of 4.5-6.5 molar equivalents of the antibody dissolved in an organic co-solvent to the diluted antibody solution, such that the volume of the organic co-solvent accounts for 5-30% of the volume of the reaction system, and then stirring and reacting the reaction system at 15-35 °C for ≥ 0.5 hours, wherein the organic co-solvent is one or more selected from the group consisting of DMA, DMSO, DMF, and ACN;
c. Hydrophobic chromatography: subjecting the antibody conjugation product obtained to purification through hydrophobic chromatography using hydrophobic filler, wherein the hydrophobic filler is Butyl Sepharose HP (GE), Capto Phenyl ImpRes (GE), Toyopearl Butyl 650M (TOSOH) or Butyl Sepharose 4 FF (GE), and the start buffer for the hydrophobic chromatography contains sodium chloride or ammonium sulfate; and

the preparation method further comprises the following step after step b or after step c:
d. Hydrolysis: displacing the antibody conjugation product into a hydrolysis buffer at pH 7.4-9.0, and heating the same therein at 25-45 °C for 2-24 hours; wherein the hydrolysis buffer comprises one or more selected from the group consisting of sodium dihydrogen phosphate, disodium hydrogen phosphate, dipotassium hydrogen phosphate, potassium dihydrogen phosphate, citric acid, glycine, tromethamine, arginine hydrochloride, hydrochloric acid, phosphoric acid, sodium hydroxide, and potassium hydroxide.

7. The preparation method according to any one of claims 1 to 6, wherein in step a:

preferably, the reducing agent is TCEP or DTT;
preferably, the reducing agent is in an amount of 6.5-10 molar equivalents of the antibody;
preferably, the reducing agent is added in a form of an aqueous solution at a concentration of 5-15 mg/mL, preferably 10 mg/mL;
preferably, the antibody is an IgG antibody, preferably IgG1 antibody;
preferably, the concentration of the antibody is 10-15 mg/mL, preferably 12 mg/mL;
preferably, the buffer is a phosphate buffer at pH 6.5-7.6.

8. The preparation method according to any one of claims 1 to 7, wherein in step b:

preferably, the buffer is at pH 7.2-7.4; preferably, the buffer is a phosphate buffer or a phosphate-EDTA buffer at pH 7.2-7.4;
preferably, when displaced, the antibody is diluted to a concentration of 3.5-10 mg/mL;
preferably, the compound represented by formula I is in an amount of 5.0-6.0 molar equivalents of the antibody;
preferably, the organic co-solvent is DMA, DMSO, or ACN;
preferably, the organic co-solvent is added to account for 6-30% of the volume of the reaction system;
preferably, the reaction system is stirred and reacted at 15-35 °C for 0.5-1 hour.

9. The preparation method according to any one of claims 1 to 8, wherein in step c: the start buffer contains ammonium sulfate, wherein the concentration of ammonium sulfate is preferably 0.45 mol/L;

preferably, the start buffer is a phosphate buffer containing ammonium sulfate, and a phosphate buffer is used as an eluent;
more preferably, the phosphate buffer is 0-100 mM disodium hydrogen phosphate - sodium dihydrogen phosphate buffer at pH 7.4-8.0.

10. The preparation method according to any one of claims 1 to 9, wherein in step d: the concentration of the antibody conjugation product in the hydrolysis buffer is 2-30 mg/mL, preferably 5-20 mg/mL;

preferably, the hydrolysis buffer is a sodium dihydrogen phosphate - disodium hydrogen phosphate buffer, a potassium dihydrogen phosphate - dipotassium hydrogen phosphate buffer or a tromethamine buffer;
preferably, the hydrolysis comprises: heating the antibody conjugation product in the hydrolysis buffer at 25-35 °C for 1-6 hours, preferably 1-3 hours;
preferably, the hydrolysis buffer is at pH 7.5-8.5, preferably pH 7.8.

**11.** A disubstituted bridged antibody-drug conjugate obtained using the preparation method according to any one of claims 1 to 10.

Figure 1

1A

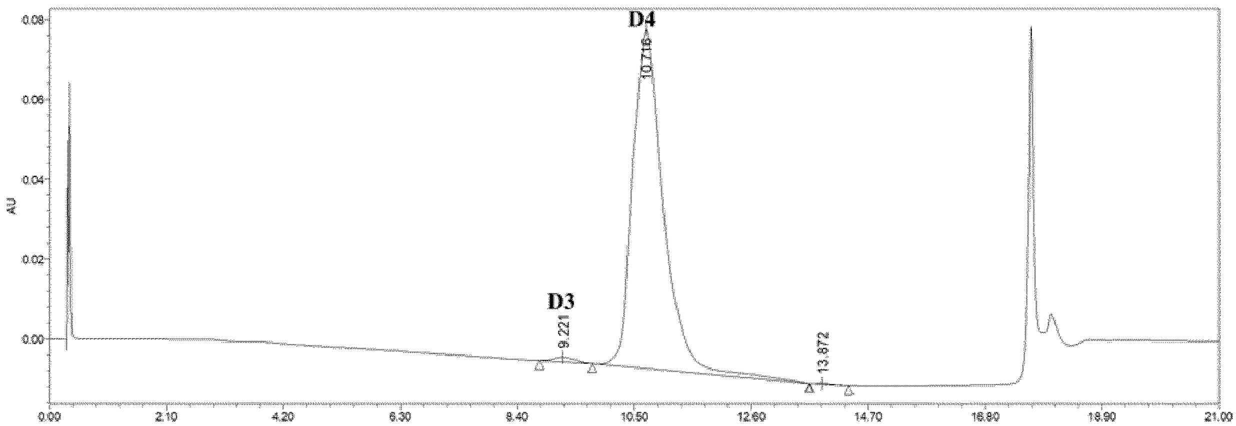


Minutes

1B

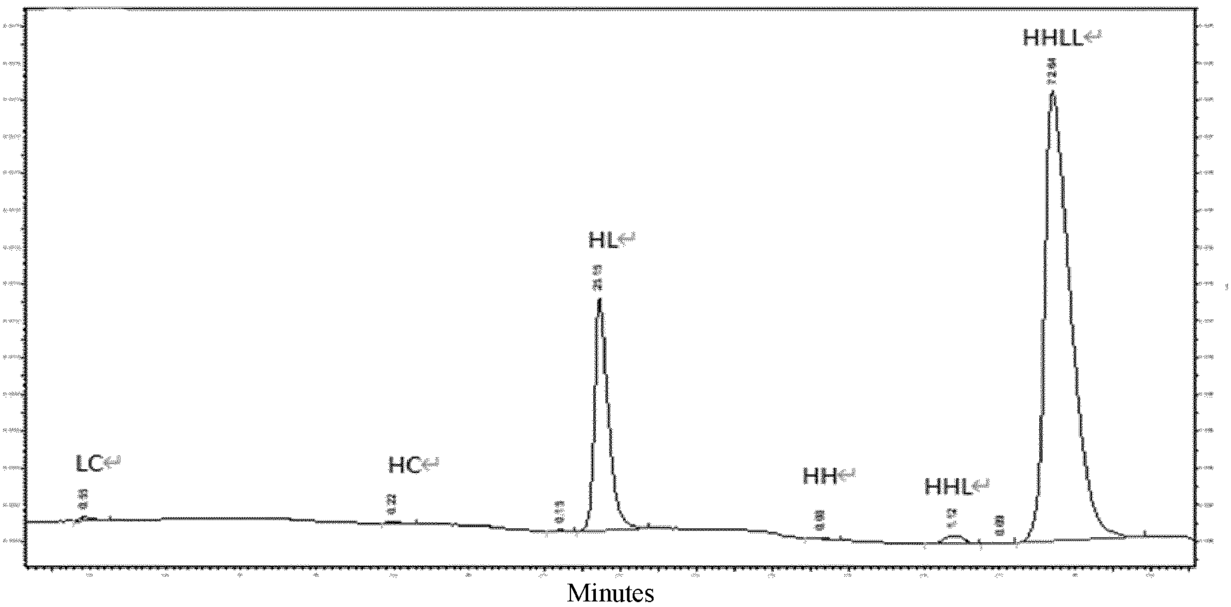


Minutes

1C

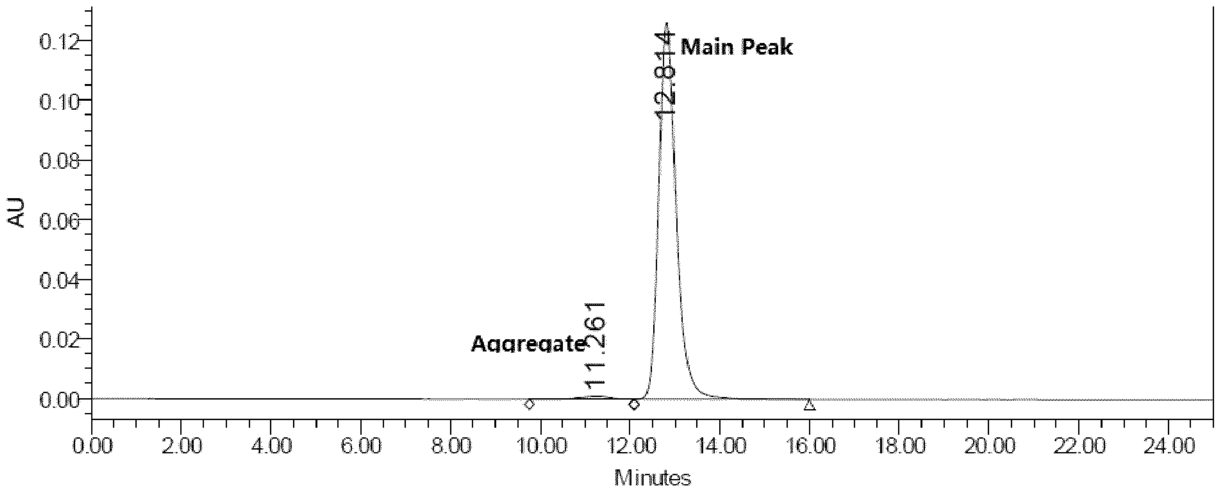


Figure 2

2A

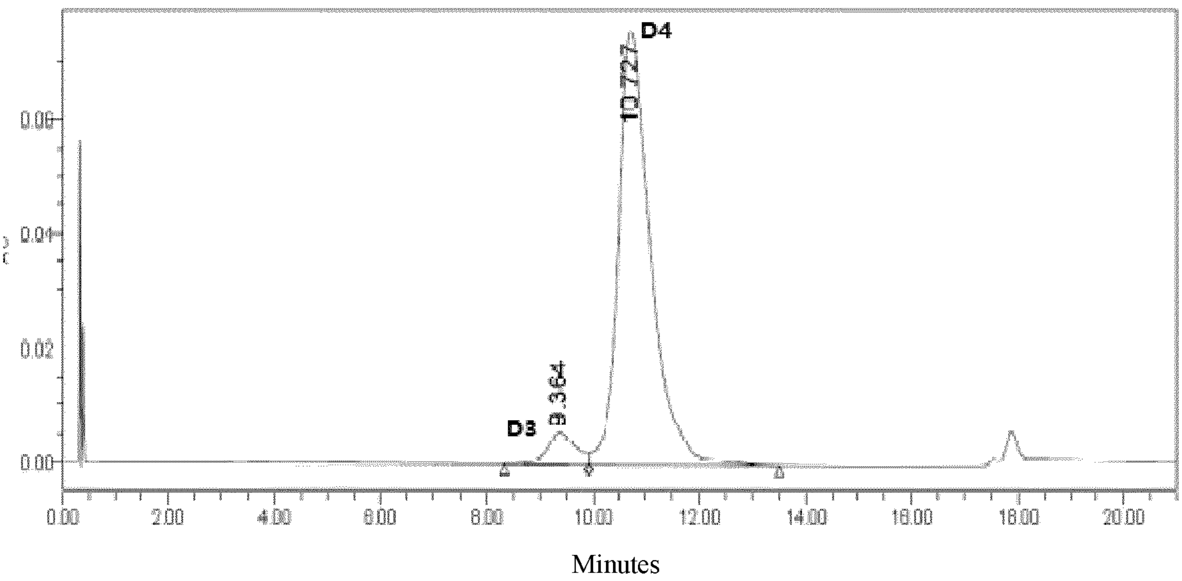

2B

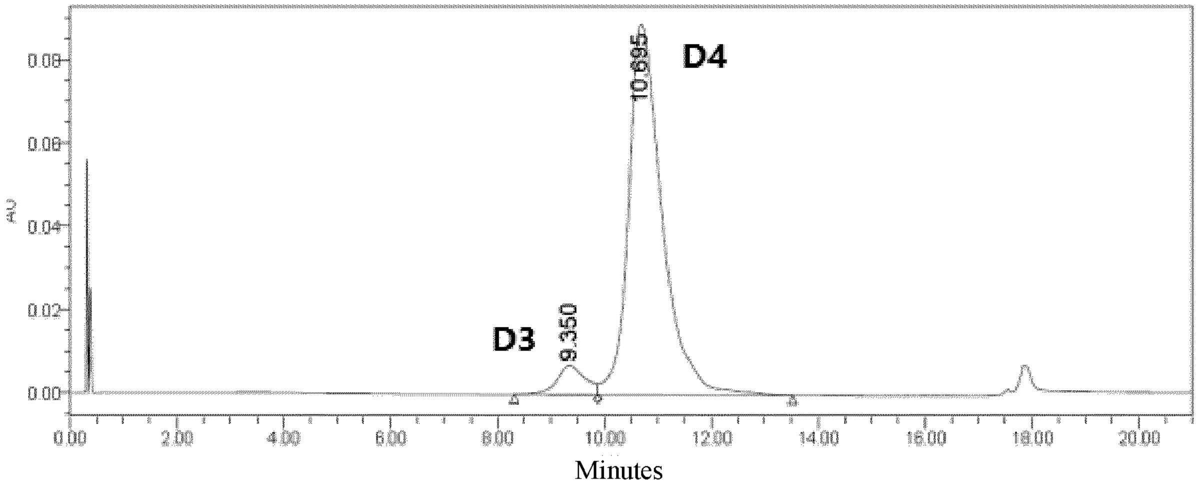
AU
0.00
0.02
0.04
D3
9.350
10.695
D4
0.00
2.00
4.00
6.00
8.00
10.00
12.00
14.00
16.00
18.00
20.00
Minutes

2C

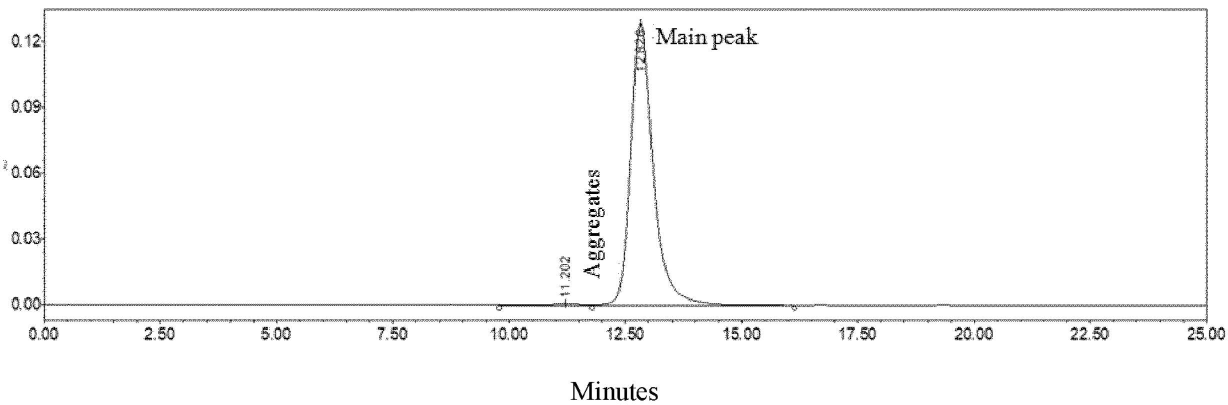
0.12
0.09
0.06
0.03
0.00
Main peak
Aggregates
11.202
0.00
2.50
5.00
7.50
10.00
12.50
15.00
17.50
20.00
22.50
25.00
Minutes

2D

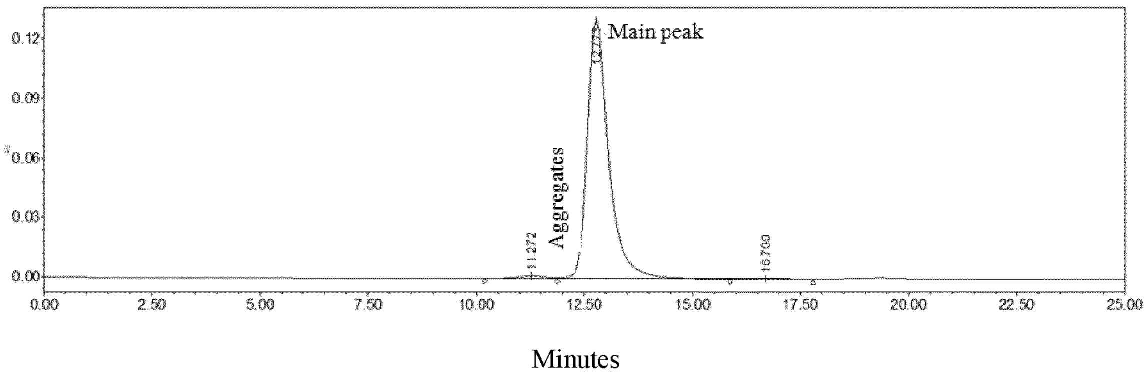
0.12
0.09
0.06
0.03
0.00
Main peak
Aggregates
11.272
16.700
0.00
2.50
5.00
7.50
10.00
12.50
15.00
17.50
20.00
22.50
25.00
Minutes

2E

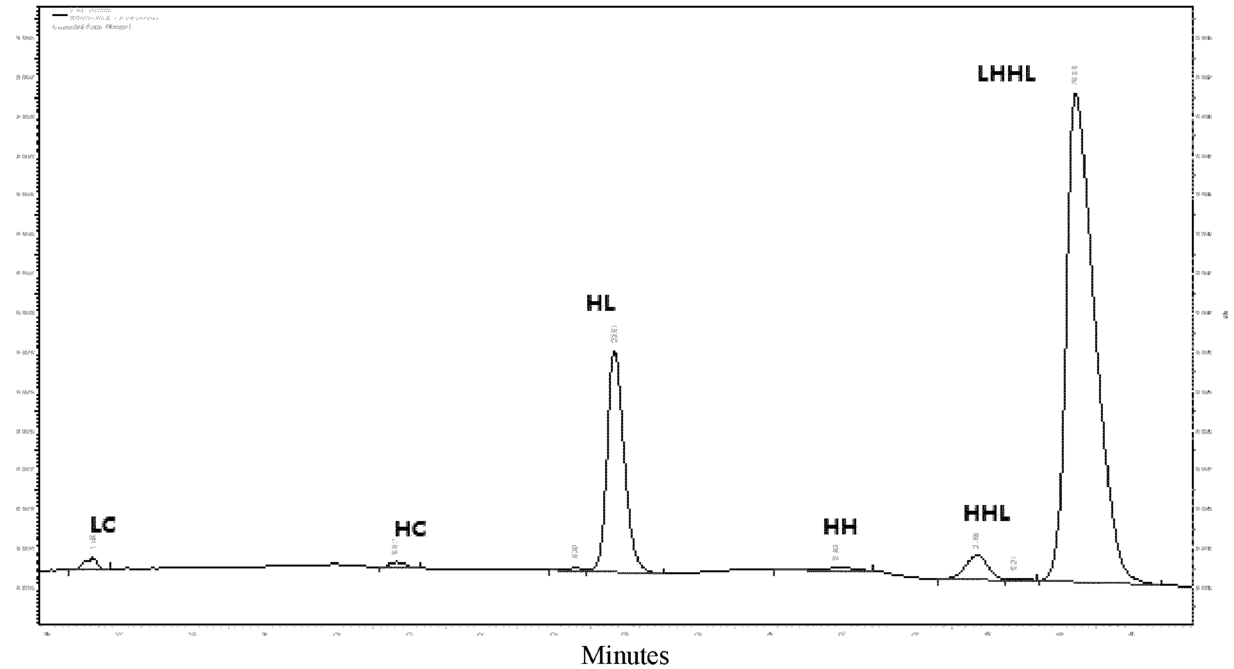


2F

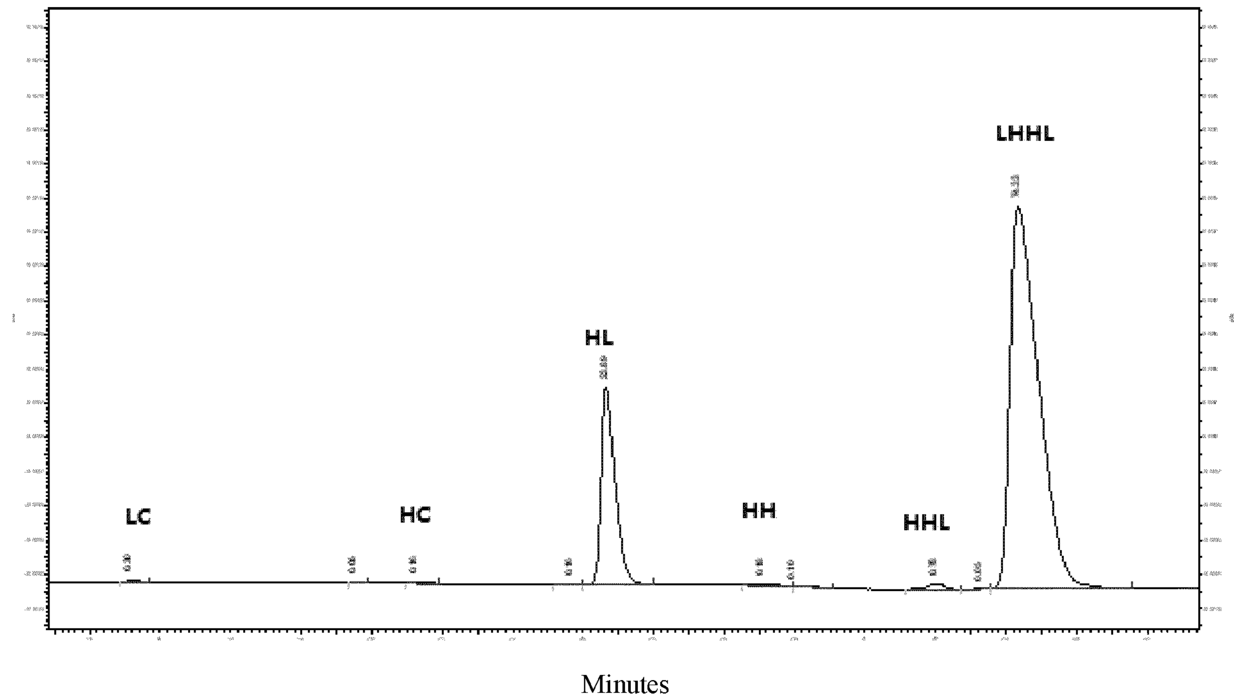

## INTERNATIONAL SEARCH REPORT

International application No.
**PCT/CN2021/121825**

**A. CLASSIFICATION OF SUBJECT MATTER**

C07D 207/456(2006.01)i; C07D 401/14(2006.01)i; A61K 31/537(2006.01)i; A61P 35/00(2006.01)i; C07K 5/027(2006.01)i; C07K 5/06(2006.01)i

According to International Patent Classification (IPC) or to both national classification and IPC

**B. FIELDS SEARCHED**

Minimum documentation searched (classification system followed by classification symbols)

C07D; A61K; A61P; C07K

Documentation searched other than minimum documentation to the extent that such documents are included in the fields searched

Electronic data base consulted during the international search (name of data base and, where practicable, search terms used)

CNABS; CNTXT; SIPOABS; DWPI; CNKI; STNext: 迈威（上海）生物科技; 周伟; 水解; 开环; 马来酰亚胺; 抗体偶联药物; hydrolysis; ring open?; antibody drug conjugate; ADC

**C. DOCUMENTS CONSIDERED TO BE RELEVANT**

| Category* | Citation of document, with indication, where appropriate, of the relevant passages | Relevant to claim No. |
|---|---|---|
| X | CN 110577600 A (SHANGHAI INSTITUTE OF MATERIA MEDICA, CHINESE ACADEMY OF SCIENCES et al.) 17 December 2019 (2019-12-17) claims 13, 24; description paragraphs 0119-0127 | 1-11 |
| X | CN 110240654 A (FUDAN UNIVERSITY) 17 September 2019 (2019-09-17) description, paragraphs 0219-0230 | 1-11 |
| X | CN 110575547 A (SHANGHAI INSTITUTE OF MATERIA MEDICA, CHINESE ACADEMY OF SCIENCES et al.) 17 December 2019 (2019-12-17) claims 1, 13; description paragraphs 0071-0079 | 1-11 |
| X | CN 110575548 A (SHANGHAI INSTITUTE OF MATERIA MEDICA, CHINESE ACADEMY OF SCIENCES) 17 December 2019 (2019-12-17) claims 1, 12; description paragraphs 0063-0071 | 1-11 |
| Y | CN 109810039 A (SHANGHAI QINGRUN PHARMACEUTICAL TECHNOLOGY CO., LTD.) 28 May 2019 (2019-05-28) claim 5; description paragraphs 0072-0083 | 1-11 |
| Y | CN 108452321 A (SICHUAN BAILI PHARMACEUTICAL CO., LTD.) 28 August 2018 (2018-08-28) description, paragraphs 0006 and 0058 | 1-11 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 December 2021** | **30 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/121825**

**C. DOCUMENTS CONSIDERED TO BE RELEVANT**

| Category* | Citation of document, with indication, where appropriate, of the relevant passages | Relevant to claim No. |
|---|---|---|
| A | WO 2013121175 A1 (UCL BUSINESS PLC) 22 August 2013 (2013-08-22)<br>entire document | 1-11 |
| A | CN 104822656 A (SEATTLE GENETICS INC.) 05 August 2015 (2015-08-05)<br>entire document | 1-11 |

Form PCT/ISA/210 (second sheet) (January 2015)

**INTERNATIONAL SEARCH REPORT**
**Information on patent family members**

International application No.
**PCT/CN2021/121825**

| Patent document cited in search report | Publication date (day/month/year) | Patent family member(s) | Publication date (day/month/year) |
|---|---|---|---|
| CN 110577600 A | 17 December 2019 | CN 110577600 B | 04 May 2021 |
| CN 110240654 A | 17 September 2019 | EP 3783025 A1 | 24 February 2021 |
| | | US 2021024646 A1 | 28 January 2021 |
| | | CN 110869393 A | 06 March 2020 |
| | | CA 3093327 A1 | 12 September 2019 |
| CN 110575547 A | 17 December 2019 | None | |
| CN 110575548 A | 17 December 2019 | None | |
| CN 109810039 A | 28 May 2019 | None | |
| CN 108452321 A | 28 August 2018 | EP 3769786 A1 | 27 January 2021 |
| | | WO 2018233571 A1 | 27 December 2018 |
| | | SG 11202007919 R A | 29 September 2020 |
| | | US 2021100912 A1 | 08 April 2021 |
| | | AU 2018288029 A1 | 21 January 2021 |
| | | WO 2018233571 A9 | 22 August 2019 |
| | | CA 3095067 A1 | 27 December 2018 |
| | | DE 112018007259 T5 | 24 December 2020 |
| WO 2013121175 A1 | 22 August 2013 | EP 2814512 A1 | 24 December 2014 |
| | | US 10010623 B2 | 03 July 2018 |
| | | US 2015037360 A1 | 05 February 2015 |
| CN 104822656 A | 05 August 2015 | CA 2869777 A1 | 21 November 2013 |
| | | MX 2014013807 A | 04 February 2015 |
| | | AU 2020202805 B2 | 17 December 2020 |
| | | NZ 724892 A | 27 April 2018 |
| | | EA 201401254 A1 | 30 September 2015 |
| | | MX 368678 B | 11 October 2019 |
| | | JP 6423340 B2 | 14 November 2018 |
| | | MX 2019012201 A | 28 November 2019 |
| | | CN 104822656 B | 01 May 2018 |
| | | SG 10201608904 Y A | 29 December 2016 |
| | | JP 2018162251 A | 18 October 2018 |
| | | NZ 741211 A | 27 September 2019 |
| | | AU 2013263002 A1 | 25 September 2014 |
| | | KR 102144069 B1 | 13 August 2020 |
| | | HK 1207388 A1 | 29 January 2016 |
| | | EP 2850094 A2 | 25 March 2015 |
| | | AU 2013263002 C1 | 31 May 2018 |
| | | JP 2015521187 A | 27 July 2015 |
| | | JP 6709247 B2 | 10 June 2020 |
| | | ZA 201407369 B | 26 February 2020 |
| | | AU 2020202805 A1 | 21 May 2020 |
| | | EP 2850094 A4 | 06 July 2016 |
| | | KR 20200097000 A | 14 August 2020 |
| | | AU 2018201190 A1 | 08 March 2018 |
| | | BR 112014028222 A2 | 27 June 2017 |
| | | JP 2020122023 A | 13 August 2020 |
| | | EA 037203 B1 | 18 February 2021 |
| | | CN 108465112 A | 31 August 2018 |
| | | WO 2013173337 A3 | 11 June 2015 |
| | | AU 2021201636 A1 | 08 April 2021 |

Form PCT/ISA/210 (patent family annex) (January 2015)

**INTERNATIONAL SEARCH REPORT**
**Information on patent family members**

International application No.
**PCT/CN2021/121825**

| Patent document cited in search report | Publication date (day/month/year) | Patent family member(s) | | | Publication date (day/month/year) |
|---|---|---|---|---|---|
| | | WO | 2013173337 | A2 | 21 November 2013 |
| | | AU | 2018201190 | B2 | 30 January 2020 |
| | | SG | 11201406252 W | A | 30 October 2014 |
| | | KR | 20150009991 | A | 27 January 2015 |
| | | AU | 2013263002 | B2 | 30 November 2017 |
| | | NZ | 630870 | A | 28 October 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202011046911X
- CN 201380025774
- CN 201310025021
- WO 2018095422 A1
- WO 20180954221 A1